(19) Europäisches Patentamt
European Patent Office
Office européen des brevets

(11) EP 1 426 038 B1

# (12) FASCICULE DE BREVET EUROPEEN

(45) Date de publication et mention de la délivrance du brevet:
**27.12.2006 Bulletin 2006/52**

(51) Int Cl.:
***A61K 8/34*** *(2006.01)* ***A61K 8/87*** *(2006.01)*
***A61K 8/898*** *(2006.01)* ***A61Q 5/10*** *(2006.01)*

(21) Numéro de dépôt: **03292932.5**

(22) Date de dépôt: **26.11.2003**

(54) **Composition de teinture d'oxydation pour fibres kératiniques comprenant un colorant d'oxydation, un polymère associatif et une silicone aminée**

Zusammensetzung zur Oxidationsfärbung von keratinischen Fasern enthaltend ein Oxidationsfarbstoff, ein assoziatives Polymer und ein aminiertes Silikon

Composition for the oxidative dyeing of keratin fibres containing an oxydation dye, a associative polymer and an amine silicone

(84) Etats contractants désignés:
**AT BE BG CH CY CZ DE DK EE ES FI FR GB GR HU IE IT LI LU MC NL PT RO SE SI SK TR**

(30) Priorité: **06.12.2002 FR 0215470**

(43) Date de publication de la demande:
**09.06.2004 Bulletin 2004/24**

(73) Titulaire: **L'ORÉAL**
**75008 Paris (FR)**

(72) Inventeurs:
- **Cottard, François**
  **92400 Courbevoie (FR)**
- **Rondeau, Christine**
  **78500 Sartrouville (FR)**

(74) Mandataire: **Casalonga, Axel**
**Bureau Casalonga & Josse**
**Bayerstrasse 71/73**
**80335 München (DE)**

(56) Documents cités:
**WO-A1-02/074273**
**WO-A2-01/72271**
**WO-A2-02/078661**
**US-A- 6 156 076**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 426 038 B1

## Description

La présente invention est relative à une composition de teinture d'oxydation des fibres kératiniques, en particulier des fibres kératiniques humaines et plus particulièrement des cheveux, comprenant au moins un colorant d'oxydation, au moins un polymère associatif et au moins une silicone aminée.

Il est connu de teindre les fibres kératiniques, et en particulier les cheveux humains, avec des compositions de teinture contenant des précurseurs de colorants d'oxydation, généralement appelés « bases d'oxydation », en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des « bases d'oxydation » sur elles-mêmes, soit d'une condensation oxydative des « bases d'oxydation » sur des composés modificateurs de coloration, ou « coupleurs », qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les « bases d'oxydation » et d'autre part par les « coupleurs », permet l'obtention d'une palette très riche en coloris.

Les compositions de teinture d'oxydation comprennent en outre généralement des polymères cationiques afin d'améliorer les propriétés cosmétiques.

Cependant, la demanderesse a constaté que les compositions tinctoriales comprenant ces polymères cationiques étaient peu stables, présentaient de propriétés cosmétiques peu satisfaisantes, et peu rémanentes aux shampooings.

Or, après d'importantes recherches menées sur la question, la demanderesse vient maintenant de découvrir que des compositions de teinture d'oxydation comprenant un colorant d'oxydation, un polymère associatif particulier et une silicone aminée particulière présentent une bonne stabilité physico-chimique, ainsi que de bonnes propriétés cosmétiques, en particulier la douceur, le lissage, la souplesse, la légèreté.

Elle a également constaté que lesdites compositions conféraient des propriétés cosmétiques rémanentes aux shampooings. Elle a ainsi observé une très bonne rémanence de la couleur aux shampooings.

Elle a également constaté que lesdites compositions permettaient d'obtenir une sélectivité faible, c'est-à-dire des écarts de coloration faibles tout au long d'une même fibre kératinique, qui peut être différemment sensibilisée (i.e. abîmée) entre sa pointe et sa racine.

La présente invention a ainsi pour objet une composition pour la teinture d'oxydation des fibres kératiniques, en particulier des fibres kératiniques humaines, telles que les cheveux, caractérisée en ce qu'elle comprend, dans un milieu approprié pour la teinture,

a) au moins un colorant d'oxydation,
b) au moins un polymère associatif cationique tel que défini en revendication 1, et
c) au moins une silicone aminée,

le rapport en poids silicone(s) aminée(s)/polymère(s) associatif(s) étant supérieur à 1.

Un autre objet de l'invention porte sur une composition prête à l'emploi pour la teinture des fibres kératiniques qui comprend au moins un colorant d'oxydation, au moins un polymère associatif cationique tel que défini dans la revendication 1, au moins une silicone aminée, le rapport en poids silicone(s) aminée(s)/polymère(s) associatif(s) étant supérieur à 1, et un agent oxydant.

Par « composition prête à l'emploi », on entend, au sens de l'invention, la composition destinée à être appliquée telle quelle sur les fibres kératiniques, c'est-à-dire qu'elle peut être stockée telle quelle avant utilisation ou résulter du mélange extemporané de deux ou plusieurs compositions.

L'invention vise également un procédé de teinture d'oxydation des fibres kératiniques, et en particulier des fibres kératiniques humaines telles que les cheveux, consistant à appliquer sur les fibres une composition (A) contenant, dans un milieu approprié pour la teinture, au moins un colorant d'oxydation, au moins un polymère associatif et au moins une silicone aminée, le rapport en poids silicone(s) aminée(s)/polymère(s) associatif(s) étant supérieur à 1, la couleur étant révélée à pH alcalin, neutre ou acide, à l'aide d'une composition (B) contenant au moins un agent oxydant, qui est mélangée juste au moment de l'emploi à la composition (A) ou qui est appliquée sur les fibres séquentiellement avant ou après la composition (A), avec ou sans rinçage intermédiaire.

L'invention a également pour objet des dispositifs de teinture à plusieurs compartiments ou « kits » à plusieurs compartiments pour la teinture d'oxydation des fibres kératiniques, en particulier des fibres kératiniques humaines telles que les cheveux. Un dispositif selon l'invention peut comporter un premier compartiment contenant au moins un colorant d'oxydation, au moins un polymère associatif et au moins une silicone aminée, le rapport en poids silicone(s) aminée(s)/polymère(s) associatif(s) étant supérieur à 1, et un deuxième compartiment contenant un agent oxydant.

D'autres caractéristiques, aspects, objets et avantages de l'invention apparaîtront encore plus clairement à la lecture de la description et des exemples qui suivent.

Les silicones aminées selon l'invention peuvent être choisies parmi les silicones aminées référencées dans le document « International Cosmetic Ingredient Dictionary and Handbook », Ninth Edition 2002 » sous les appellations Amodimethicone et Trimethylsiloxyamadimethicone.

Les silicones aminées selon l'invention sont de préférence choisies parmi les silicones aminées de formules (I), (II) ou (III) :

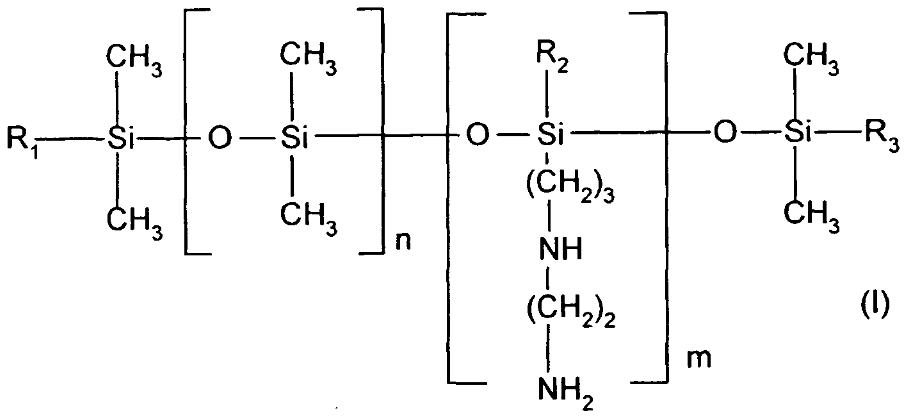

(I)

dans laquelle :

m et n sont des nombres tels que la somme (n+m) peut varier notamment de 1 à 1000 et en particulier de 50 à 250 et plus particulièrement de 100 à 200,

n pouvant désigner un nombre de 0 à 999 et notamment de 49 à 249 et plus particulièrement de 125 à 175 et m pouvant désigner un nombre de 1 à 1000, et notamment de 1 à 10 et plus particulièrement de 1 à 5 ;

$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence, le radical alcoxy est un radical méthoxy.

Le rapport molaire hydroxy/alcoxy est de préférence compris entre 0,2 :1 et 0,4 :1 et de préférence entre 0,25 : 1 et 0,35 :1 et plus particulièrement est égal à 0,3.

La silicone aminée de formule (I) présente une masse moléculaire moyenne en poids allant de préférence de 2000 à 1000000 et encore plus particulièrement de 3500 à 200000.

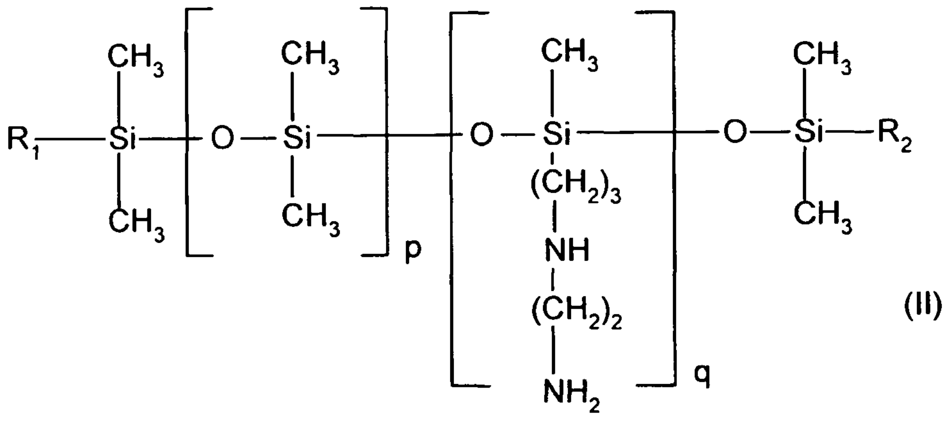

(II)

dans laquelle :

p et q sont des nombres tels que la somme (p+q) peut varier notamment de 1 à 1000 et en particulier de 50 à 350 et plus particulièrement de 150 à 250,
p pouvant désigner un nombre de 0 à 999 et notamment de 49 à 349 et plus particulièrement de 159 à 239 et q pouvant désigner un nombre de 1 à 1000, et notamment de 1 à 10 et plus particulièrement de 1 à 5 ;
$R_1$, $R_2$, différents, représentent un radical hydroxy ou alcoxy en $C_1$-$C_4$, l'un au moins des radicaux $R_1$ ou $R_2$ désignant un radical alcoxy.

De préférence, le radical alcoxy est un radical méthoxy.

Le rapport molaire hydroxy/alcoxy est de préférence compris entre 1 :0,8 et 1 :1,1 et de préférence entre 1 : 0,9 et 1 :1 et plus particulièrement est égal à 1 :0,95.

La silicone aminée de formule (II) présente une masse moléculaire moyenne en poids allant de préférence de 2000 à 200.000 et encore plus particulièrement de 5.000 à 100.000 et plus particulièrement encore de 10.000 à 50.000.

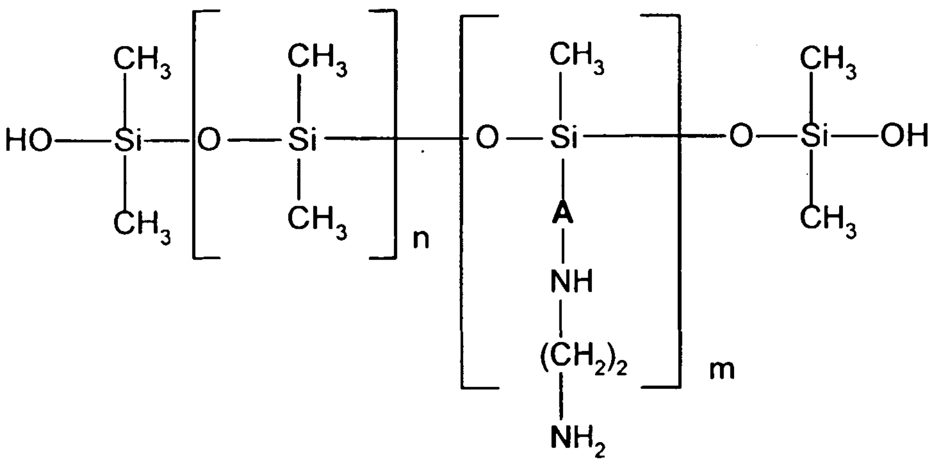

(III)

dans laquelle :

A désigne un radical alkylène linéaire ou ramifié en $C_4$-$C_8$ et de préférence en $C_4$,
m et n sont des nombres tels que la somme (n+m) peut varier notamment de 1 à 2000 et en particulier de 50 à 150, n pouvant désigner un nombre allant de 0 à 1999 et notamment de 49 à 149 et m pouvant désigner un nombre allant de 1 à 2000, et notamment de 1 à 10.

La viscosité de la silicone de formule (III) est de préférence supérieure à 25000 $mm^2/s$ à 25°C. Plus préférentiellement, cette viscosité peut aller de 30 000 à 200 000 $mm^2/s$ à 25°C et encore plus préférentiellement de 30 000 à 150 000 $mm^2/s$ à 25°C. la viscosité des silicones est par exemple mesurée selon la norme « ASTM 445 Appendice C ».

La silicone aminée de formule (III) présente une masse moléculaire allant de préférence de 2000 à 1000000 et encore plus particulièrement de 3500 à 200000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. on injecte 200 μl d'une solution à 0,5% en poids en moyenne dans le THF. La détection se fait par réfractométrie et UV métrie.

Les produits commerciaux correspondant à ces silicones de formules (I) à (III) peuvent inclure dans leur composition une ou plusieurs autres silicones aminées dont la structure est différente des structures (I) à (III).

Un produit contenant des silicones aminées de structure (I) est proposé par la société WACKER sous la dénomination BELSIL ADM 652®

Des produits contenant des silicones aminées de structure (II) sont proposés par la société WACKER sous la dénomination Fluid WR 1300® et BELSIL ADM 6057®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensio-actifs peuvent être de toute nature, mais de préférence cationique et/ou non ionique.

Les particules de silicone dans l'émulsion ont une taille moyenne allant généralement de 3 à 500 nm.

De préférence, pour les silicones aminées de formule (II), on utilise des microémulsions de taille allant de 5 à 60 nm et plus particulièrement de 10 à 50 nm.

On peut utiliser selon l'invention les microémulsions de silicones aminées de formule (II) proposées sous la dénomination FINISH CT 96 E® ou SLM 28020® par la société WACKER.

Les particules de silicone aminées de formule (III) dans l'émulsion ont une taille moyenne allant de préférence de 5 à 300 nm, plus particulièrement de 10 à 275 nm et encore plus particulièrement de 150 à 275 nm.

Une silicone de formule (III) est par exemple la DC-28299® de la société CORNING.

De préférence, la silicone aminée de formule (I) ou (II) est choisie de telle façon que l'angle de contact d'un cheveu traité avec une composition contenant de l'eau et 2% MA (matières actives) de ladite silicone selon l'invention soit compris entre 90 et 180° et de préférence entre 90 et 130°, bornes incluses.

De préférence, la composition contenant la ou les silicones aminées de formule (I) ou (II) est telle que l'angle de contact d'un cheveu traité avec ladite composition est compris entre 90 et 180° de préférence entre 90 et 130° bornes incluses.

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°, bornes incluses.

Le test s'effectue avec des mèches de cheveux naturels ayant été décolorées dans les mêmes conditions puis lavées.

Chaque mèche de 1g est placée dans un cristallisoir de 75 mm de diamètre puis recouverte de façon homogène par 5 ml de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré via une observation microscopique.

La force moyenne de mouillabilité sur 10 cheveux et la section des cheveux analysés permettent d'obtenir l'angle de contact du cheveu sur l'eau, selon la formule :

$$F = P*\Gamma lv*\cos\theta$$

Où F est la force de mouillabilité exprimée en Newton, P le périmètre du cheveu en mètre, Γlv la tension interfaciale liquide/vapeur d'eau en $J/m^2$ et θ l'angle de contact.

Le produit SLM 28020® de WACKER à 12% dans l'eau (soit 2% en silicone aminée) conduit à un angle de contact de 93° selon le test indiqué ci-dessus.

La silicone aminée de formule (I), (II) ou (III) est présente dans la composition en une quantité allant de préférence de 0,1 à 10%, et de préférence de 0,5 à 5% en poids du poids total de la composition.

Les polymères associatifs sont des polymères dont les molécules sont capables, dans le milieu de formulation, de s'associer entre elles ou avec des molécules d'autres composés.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes (comprenant au moins une chaîne grasse) par lesquelles les polymères interagissent et se rassemblent entre eux ou avec d'autres molécules.

Les polymères associatifs selon l'invention sont choisis parmi les polymères associatifs, cationiques comportant au moins une chaîne grasse.

La chaîne grasse comporte de préférence de 8 à 30 atomes de carbone, et encore plus préférentiellement de 10 à 30 atomes de carbone.

Les polymères associatifs à chaîne grasse de type cationique utilisés dans la présente invention sont choisis parmi les dérivés de cellulose quaternisée, et, les polyuréthanes cationiques.

Les dérivés de cellulose quaternisée sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en $C_8$-$C_{30}$, les hydroxyéthylcelluloses quaternisées modifiées par un groupement alkyle en $C_{12}$ ou $C_{18}$ tels que les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA.

Les polyuréthanes associatifs cationiques selon la présente invention sont plus particulièrement choisis parmi les polyuréthanes amphiphiles associatifs cationiques, hydrosolubles ou hydrodispersibles.

Le terme "hydrosoluble" ou "soluble dans l'eau" concernant les polyuréthanes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.

On entend par polyuréthanes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

La famille de polyuréthanes amphiphiles cationiques selon l'invention a été décrite par la demanderesse dans la demande de brevet français N°-0009609; elle peut être représentée par la formule générale (Ia) suivante :

$$R\text{-}X\text{-}(P)_n\text{-}[L\text{-}(Y)_m]_r\text{-}L'\text{-}(P')_p\text{-}X'\text{-}R \qquad (Ia)$$

dans laquelle :

R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;

la molécule contenant au moins une fonction amine protonée ou quaternisée et au moins un groupement hydrophobe.

Dans un mode de réalisation préféré des polyuréthanes de la présente invention, les seuls groupements

hydrophobes sont les groupes R et R' aux extrémités de chaîne.

Une famille préférée de polyuréthanes amphiphiles cationiques est celle correspondant à la formule (Ia) décrite ci-dessus et dans laquelle :

R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes amphiphiles cationiques est celle correspondant à la formule (Ia) ci-dessus dans laquelle:

R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes amphiphiles cationiques est celle correspondant à la formule (Ia) ci-dessus dans laquelle:

R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofunctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes :

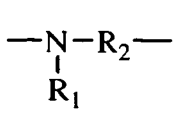
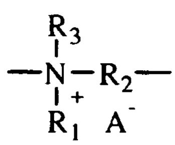
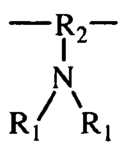
ou
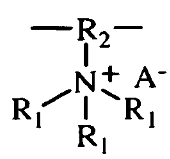
pour X

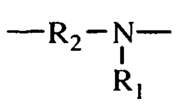 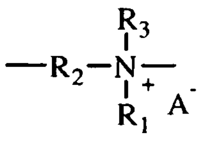 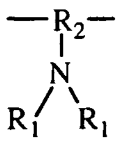 ou 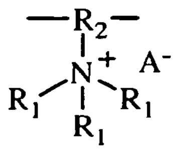 pour X'

dans lesquelles :

$R_2$ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;

$R_1$ et $R_3$, identiques ou différents, désignent un radical alkyle ou alcényle en $C_1$-$C_{30}$, linéaire ou ramifié, un radical aryle, l'un au moins des atomes de carbone pouvant être remplacé par un hétéroatome choisi parmi N, S, O, P ;

A est un contre-ion physiologiquement acceptable.

Les groupements L, L' et L" représentent un groupe de formule :

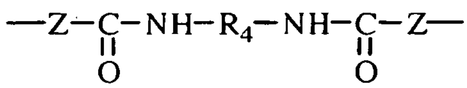

dans laquelle :

Z représente -O-, -S- ou -NH- ; et

$R_4$ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes :

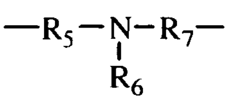 ou 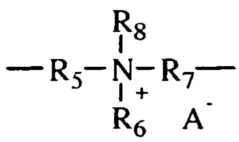

ou 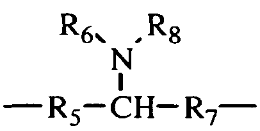 ou 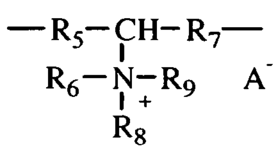

ou 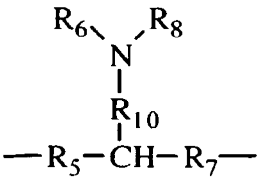 ou 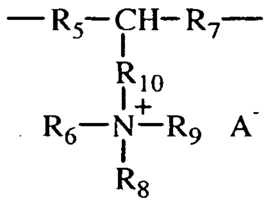

dans lesquelles :

$R_5$ et $R_7$ ont les mêmes significations que $R_2$ défini précédemment;
$R_6$, $R_8$ et $R_9$ ont les mêmes significations que $R_1$ et $R_3$ définis précédemment ;
$R_{10}$ représente un groupe alkylène, linéaire ou ramifié, éventuellement insaturé et pouvant contenir un ou plusieurs hétéroatomes choisis parmi N, O, S et P,
et A est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly (oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (Ia) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.
Ce type de composés peut être représenté par l'une des formules suivantes :

$$HZ-(P)_n-ZH,$$

ou

$$HZ-(P')_p-ZH$$

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (Ia) est un diisocyanate correspondant à la formule :

$$O=C=N-R_4-N=C=O$$

dans laquelle $R_4$ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanedii socyanate.

Un troisième composé entrant dans la préparation du polyuréthane de formule (Ia) est un composé hydrophobe destiné à former les groupes hydrophobes terminaux du polymère de formule (Ia).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné-hydroxyle.

Le groupe hydrophobe du polyuréthane de formule (Ia) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (Ia) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.

Lesdits polyuréthanes associatifs cationiques sont hydrosolubles ou hydrodispersibles.

Le ou les polymères associatifs sont présents dans la composition à des teneurs en poids comprises de préférence entre 0,05 et 10%, et encore de préférence entre 0,1 et 5% du poids total de la composition.

Le rapport en poids de la silicone aminée sur le polymère associatif est de préférence compris entre 0,1 et 10, et plus préférentiellement entre 0,5 et 5.

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques, ainsi que leurs sels d'addition avec un acide.

On peut notamment citer :

- (I) les paraphénylènediamines de formule (I) suivante et leurs sels d'addition avec un acide :

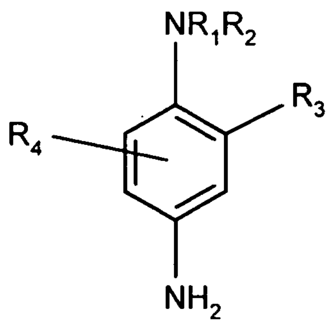

**(I)**

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$ alcoxy($C_1$-$C_4$)alkyle($C_1$-$C_4$), alkyle en $C_1$-$C_4$ substitué par un groupement azoté, phényle ou 4'-aminophényle ;
$R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
$R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido;
$R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
$R_4$ représente un atome d'hydrogène, d'halogène ou un radical alkyle en C,-$C_4$.

Parmi les groupements azotés de la formule (I) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl) amino-2-méthylaniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-l-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

- (II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide :

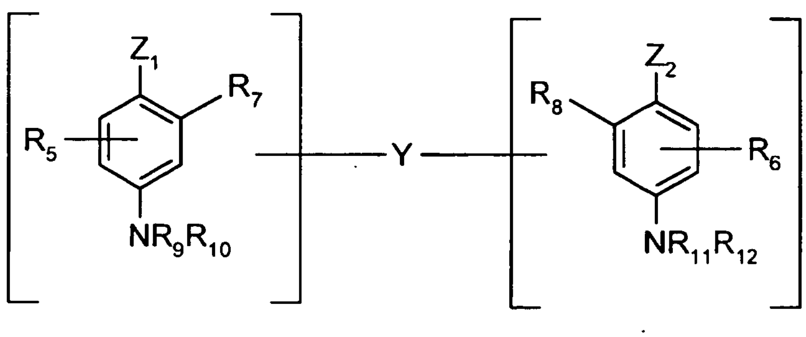

(II)

dans laquelle :

- $Z_1$ et $Z_2$, identiques ou différents, représentent un radical hydroxyle ou - $NH_2$ pouvant être substitué par un radical alkyle en $C_1$-$C_4$ ou par un bras de liaison Y ;

- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diaminopropanol, le 1,8-bis-(2,5-diaminophénoxy)-3,5-dioxaoctane ou l'un de leurs sels d'addition avec un acide sont particulièrement préférés.

- (III) les para-aminophénols répondant à la formule (III) suivante, et leurs sels d'addition avec un acide :

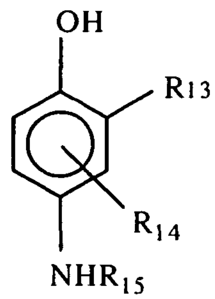

(III)

dans laquelle :

$R_{13}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

$R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$) alkyle($C_1$-$C_4$).

$R_{15}$ représente un atome d'hydrogène ou un radical alkyle en $C_1$-$C_4$.

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les

dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3.5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(é-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition (A) et/ou la composition (B) peuvent en outre plus particulièrement contenir, au moins un polymère substantif amphotère ou cationique, différent des polymères associatifs de l'invention et non siliconé.

Au sens de la présente invention, l'expression "polymère cationique" désigne tout polymère contenant des

groupements cationiques et/ou des groupements ionisables en groupements cationiques.

Les polymères substantifs cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer

(1) Les homopolymères ou copolymères dérivés d'esters ou d'amides acryliques ou méthacryliques et comportant au moins un des motifs de formules (I), (II), (III) ou (IV) suivantes:

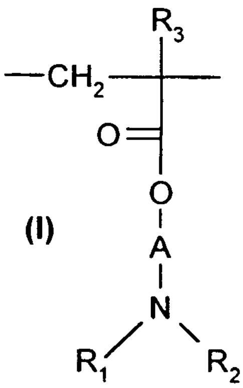
(I)

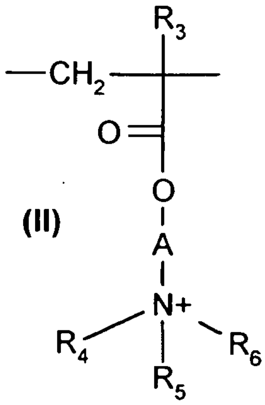
(II)

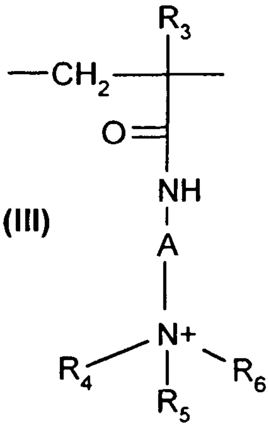
(III)

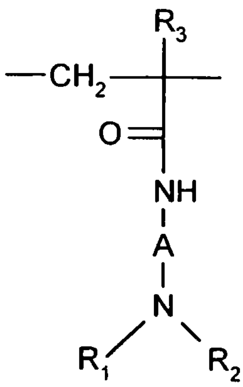
(IV)

dans lesquelles:

$R_3$ , identiques ou différents, désignent un atome d'hydrogène ou un radical $CH_3$;

A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;

$R_4$, $R_5$, $R_6$, identiques ou différents, représentent un groupe alkyle ayant de 1 à 6 atomes de carbone;

$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyl-triméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylamino-propyle quaternisés tel que le produit vendu sous la dénomination "GAFQUAT HS 100" par la société ISP.

(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium. Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

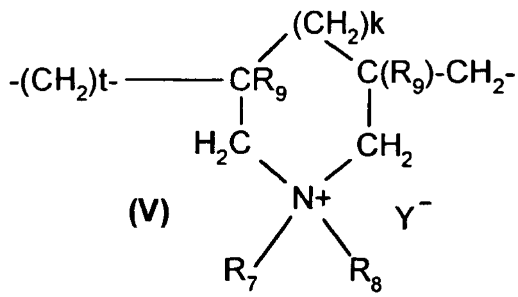
(V)

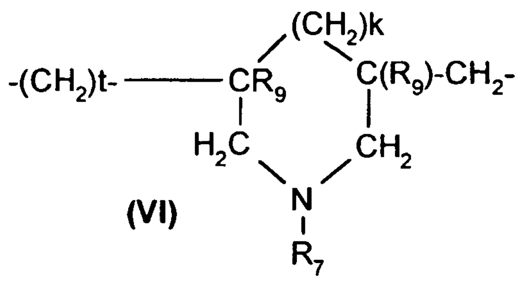
(VI)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$

indépendamment l'un de l'autre désignent de préférence un groupement alkyle ayant de 1 à 4 atomes de carbone ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. Parmi les polymères définis ci-dessus, on peut citer plus particulièrement l'homopolymère de chlorure de diméthyl-diallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

(10) Le polymère de diammonium quaternaire contenant des motifs récurrents répondant à la formule :

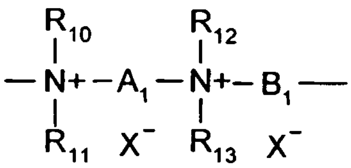

**(VII)**

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 6 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH- $R_{14}$-D où $R_{14}$ est un alkylène et D un groupement ammonium quaternaire ;
A1 et B1 représentent des groupements polyméthyléniques contenant de 2 à 6 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
Al, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement $-(CH_2)n-CO-D-OC-(CH_2)n-$ dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- .

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante:

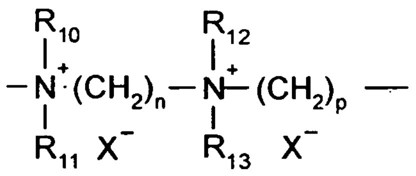

**(VIII)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

(11) Les polymères de polyammonium quaternaire constitués de motifs de formule (IX) :

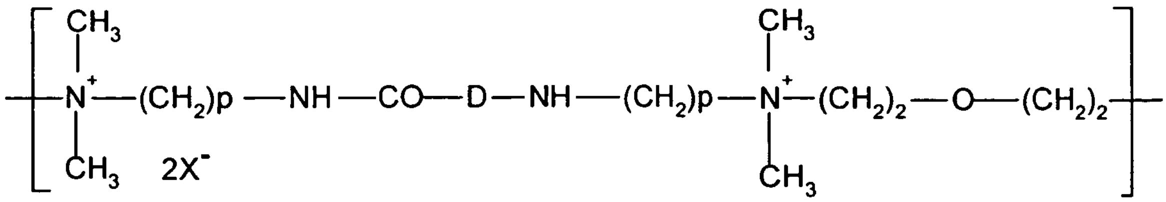

**(IX)**

dans laquelle :

p désigne un nombre entier variant de 1 à 6 environ,
D peut être nul ou peut représenter un groupement $-(CH_2)_r-CO-$ dans lequel r désigne un nombre égal à 4 ou à 7, et
X- est un anion dérivé d'un acide minéral ou organique.
Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.
Parmi ces polymères, on préfère ceux de masse moléculaire mesurée par RMN du Carbone 13 inférieure à 100000, et dans la formule de laquelle :
p est égal à 3, et,

a) D représente un groupement $-(CH_2)_4-CO-$, X désigne un atome de chlore, la masse moléculaire mesurée par RMN du Carbone 13 ($RMN^{13}C$) étant d'environ 5600 ; un polymère de ce type est proposé par la société MIRANOL sous le nom de MIRAPOL-AD1,
b) D représente un groupement $-(CH_2)_7-CO-$, X désigne un atome de chlore, la masse moléculaire mesurée par RMN du Carbone 13 ( $RMN^{13}C$) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ($RMN^{13}C$) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9,
(masse moléculaire $RMN^{13}C$, environ 7800) MIRAPOL-175, (masse moléculaire $RMN^{13}C$, environ 8000) MIRAPOL-95, (masse moléculaire $RMN^{13}C$, environ 12500).

Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13, ($RMN^{13}C$) étant d'environ 25500.

(12) Les polymères quaternaires de vinylpyrrolidone et de vinylimidazole tels que par exemple les produits commercialisés sous les dénominations Luviquat FC 905, FC 550 et FC 370 par la société B.A.S.F.

(13) Les polyamines comme le Polyquart H vendu par HENKEL, référencé sous le nom de " POLYETHYLENEGLYCOL (15) TALLOW POLYAMINE " dans le dictionnaire CTFA.

(14) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes :

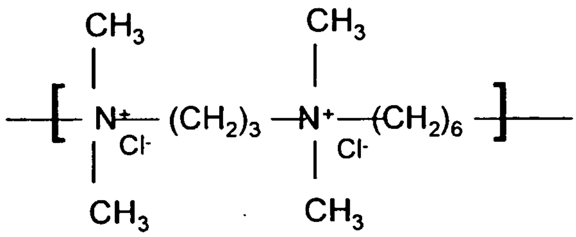

**(W)**

et notamment ceux dont le poids moléculaire, déterminé par chromatographie par perméation de gel, est compris entre 9500 et 9900;

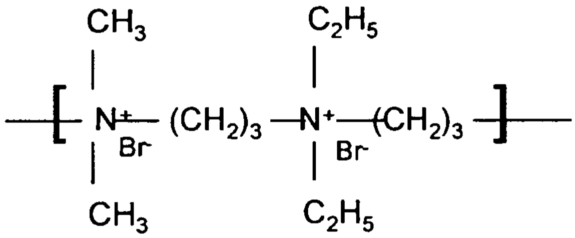

**(U)**

et notamment ceux dont le poids moléculaire, déterminé par chromatographie par perméation de gel, est d'environ 1200.
La concentration en polymère substantif cationique dans la composition selon la présente invention peut varier de 0,01 à 10% en poids par rapport au poids total de la composition, de préférence de 0,05 à 5% et plus préférentiellement encore de 0,1 à 3%.

Polymères amphotères

Les polymères substantifs amphotères utilisables conformément à la présente invention peuvent être choisis parmi les polymères comportant des motifs K et M répartis statistiquement dans la chaîne polymère, où K désigne un

motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 6 atomes de carbone et plus particulièrement le N-éthylacrylamide, le N-tertiobutyl-acrylamide, ainsi que les méthacrylamides correspondants.
Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
Les comonomères basiques préférés sont des méthacrylates d'aminoéthyle, de butyl aminoéthyle, de N,N'-diméthylaminoéthyle, de N-tertio-butyl-aminoéthyle.
On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\left[ CO\text{-}R_{19}\text{-}CO\text{-}Z \right] \qquad (X)$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

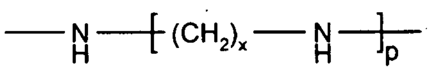 **(XI)**

où x=2 et p=2 ou 3, ou bien x=3 et p=2
ce radical dérivant de la diéthylène triamine, de la triéthylène tétraamine ou de la dipropylène triamine;
b) dans les proportions de 0 à 40 moles % le radical (XI) ci-dessus,
dans lequel x=2 et p=1 et qui dérive de l'éthylènediamine, ou le radical dérivant de la pipérazine :

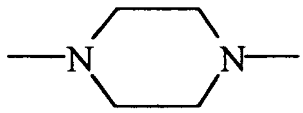

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels. Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

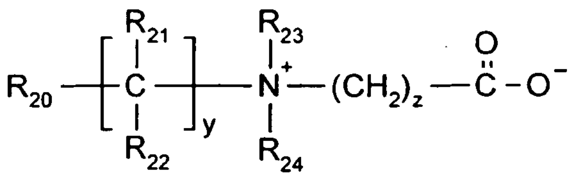 **(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne:

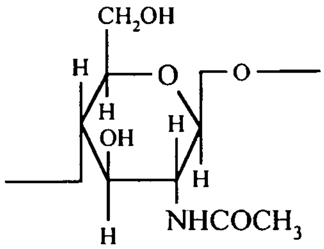

**(XIII)**

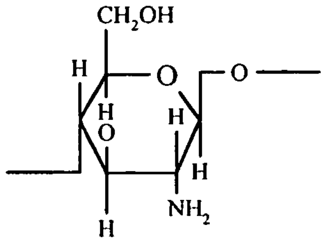

**(XIV)**

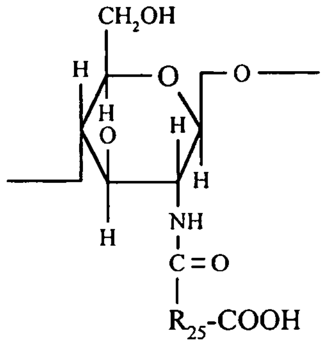

**(XV)**

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule :

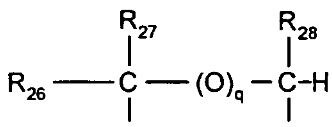

dans laquelle q désigne zéro ou 1 ;
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.
Des polymères de ce type plus particulièrement préférés comportent de 0 à 20% en poids de motifs (XIII), de 40 à 50% en poids de motifs (XIV), et de 40 à 50% en poids de motifs (XV) dans lequel $R_{25}$ désigne le radical $-CH_2-CH_2-$ ;

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exemple dans le brevet français 1 400 366 :

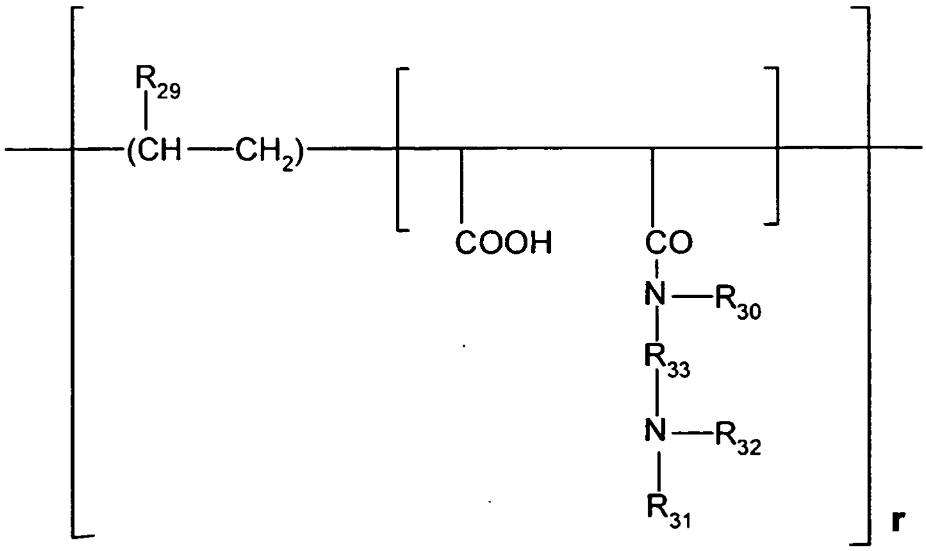

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $R_{33}$-$N(R_{31})_2$, $R_{33}$ représentant un groupement $-CH_2-CH_2-$ , $-CH_2-CH_2-CH_2-$ , $-CH_2-CH(CH_3)-$ , $R_{31}$ ayant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- (XVII)

où D désigne un radical

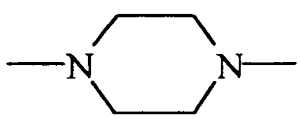

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- (XVIII)

où D désigne un radical

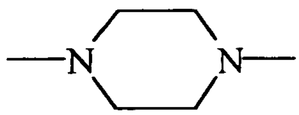

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères substantifs amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.

Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl ($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30

moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-}CONHCH_2CH_2\text{ -}N(R_3)(R_4)(CH_2COO^-)$$

dans laquelle : $R_2$ désigne un radical alkyle linéiare ou ramifié en $C_5$-$C_{20}$ provenant par exemple d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_2'\text{-}CONHCH_2CH_2\text{-}N(B)(C)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - $SO_3H$
$R_2$' désigne un radical alkyle linéaire ou ramifié, saturé ou non, en $C_5$-$C_{20}$, d'un acide $R_9$ -COOH présent par exemple dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid. A titre d'exemple on peut citer le cocoamphodiacetate commercialisé sous la dénomination commerciale MIRANOL® C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également contenir des agents d'ajustement de la rhéologie non associatifs tels que les épaississants cellulosiques
(hydroxyéthycellulose, hydroxypropylcellulose, carboxyméthylcellulose..), la gomme de guar et ses dérivés(hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.

Ces épaississants d'appoint peuvent représenter de 0,01 à 10% en poids du poids total de la composition.

Le milieu de la composition approprié pour la teinture, est de préférence un milieu aqueux constitué par de

l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition (A) peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition (B), l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale (A) et de la composition oxydante (B), est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (XIX) suivante :

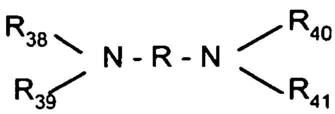

**(XIX)**

dans laquelle R est un reste propylène éventuellement substitué par un groupement hydroxyle ou un radical alkyle en $C_1$-$C_4$ ; $R_{38}$, $R_{39}$, $R_{40}$ et $R_{41}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes, et plus préférentiellement de 10 à 45 minutes, à rincer les fibres, puis éventuellement à les laver au shampooing, puis à les rincer à nouveau, et à les sécher.

Une variante de ce procédé consiste à appliquer séquentiellement de manière décalée ou simultanée sur les fibres kératiniques sèches ou humides avec un éventuel rinçage intermédiaire une composition décrite ci-dessus et une composition comprenant un agent oxydant et à laisser agir lesdites compositions pendant un temps de pose variant de

1 à 60 minutes puis à rincer les fibres, puis éventuellement à les laver au shampooing puis à les rincer à nouveau et à les sécher.

L'exemple suivant est destiné à illustrer l'invention.

On a préparé la composition suivante (quantités exprimées en pourcentages en poids)

| | |
|---|---|
| Mélange d'alcools linéaires en C18 à C24 (C18/C20/C22/C24 : 7/57/30/6 -teneur en alcool> 95%) | 3 |
| Alcool stéarylique oxyéthyléné (2OE) | 4,5 |
| Alcool stéarylique oxyéthyléné (21 OE) | 1,75 |
| Acide oléique | 2,6 |
| Polyuréthane cationique à chaîne grasse obtenu à partir de la polycondensation de 1,3bis (isocyanatométhylcyclohexane), de N,N-diméthyléthanolamine quaternisée avec le bromododécane, de N,N-diméthyléthanolamine, et de polyoxyéthylène de poids moléculaire 10000 | 0,2 |
| Acide polycrylique réticulé (produit commercialisé sous la dénomination Carbopol 980 par la société Novéon) | 0,4 |
| Hydroxypropyl méthyl cellulose | 0,2 |
| Monoisopropanolamide d'acides de coprah | 3 |
| Silicone aminée (produit commercialisé sous la dénomination SLM 28020 par la société WACKER) | 3 (en matières actives) |
| Propylène glycol | 2 |
| Métabisulfite de sodium | 0,71 |
| EDTA (acide éthylènediamine tétra-acétique) | 0,2 |
| Ter-butyl hydroquinone | 0,3 |
| 1,4-diaminobenzène | 0,2 |
| Para-aminophénol | 1,2 |
| 1,3-dihydroxybenzène | 0,1 |
| 1-hydroxy-3-amino-benzène | 0,2 |
| 1-méthyl-2-hydroxy-4-β-hydroxyéthylamino-benzène | 0,8 |
| Monoéthanolami ne | 1 |
| Ammoniaque à 20% de $NH_3$ | 11 |
| Parfum q.s | |
| Eau déminéralisée q.s.p | 100 |

On peut également employer comme silicone aminée le produit commercialisé sous la dénomination BELSLADM 652 par la société WACKER ou DC2-8 299 par la société DOW CONING.

Cette composition est mélangée au moment de l'emploi à une composition oxydante sous forme d'émulsion contenant comme agent oxydant 7,5% de peroxyde d'hydrogène à raison de 1 partie en poids de composition colorante pour 1,5 parties en poids de composition oxydante. On applique le mélange obtenu sur des mèches de cheveux naturels à 90% de blancs et on laisse poser 30 minutes. Après rinçage, lavage au shampooing et séchage, on obtient des cheveux teints dans une nuance soutenue châtain clair rouge cuivré.

## Revendications

1. Composition pour la teinture d'oxydation des fibres kératiniques, en particulier des fibres kératiniques humaines, telles que les cheveux, **caractérisée en ce qu'**elle comprend, dans un milieu approprié pour la teinture,

a) au moins un colorant d'oxydation,
b) au moins un polymère associatif cationique comportant au moins une chaîne grasse, choisi parmi :

(i) les celluloses quaternisées modifiées par des groupements comportant au moins une chaîne grasse,
(ii) les hydroxyéthylcelluloses quaternisées modifiées par des groupements comportant au moins une chaîne grasse, et
(iii) les polyuréthanes cationiques,

et
c) au moins une silicone aminée,

le rapport en poids silicone(s) aminée(s)/polymère(s) associatif(s) étant supérieur à 1.

**2.** Composition selon la revendication 1, **caractérisée en ce que** la silicone aminée est de formule (I), (II) ou (III) suivantes :

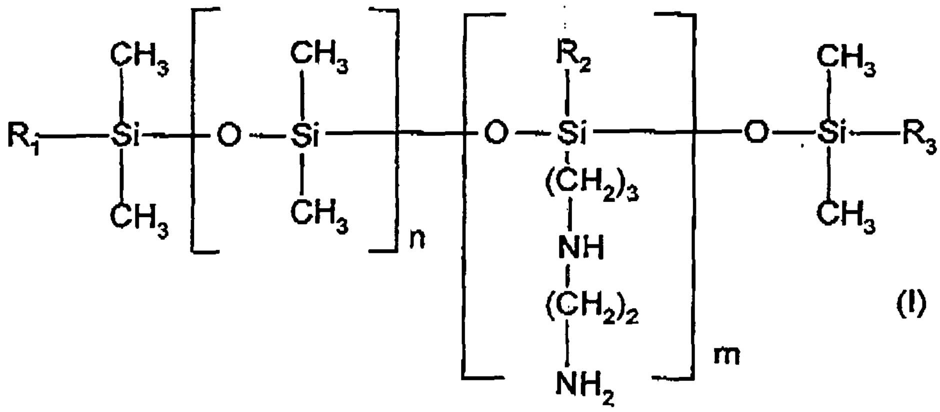

(I)

formule (I) dans laquelle :

m et n sont des nombres tels que la somme (n+m) varie de 1 à 1000 et en particulier de 50 à 250 et plus particulièrement de 100 à 200,
n désignant un nombre de 0 à 999 et notamment de 49 à 249 et plus particulièrement de 125 à 175 et m désignant un nombre de 1 à 1000, et notamment de 1 à 10 et plus particulièrement de 1 à 5 ;
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy ;

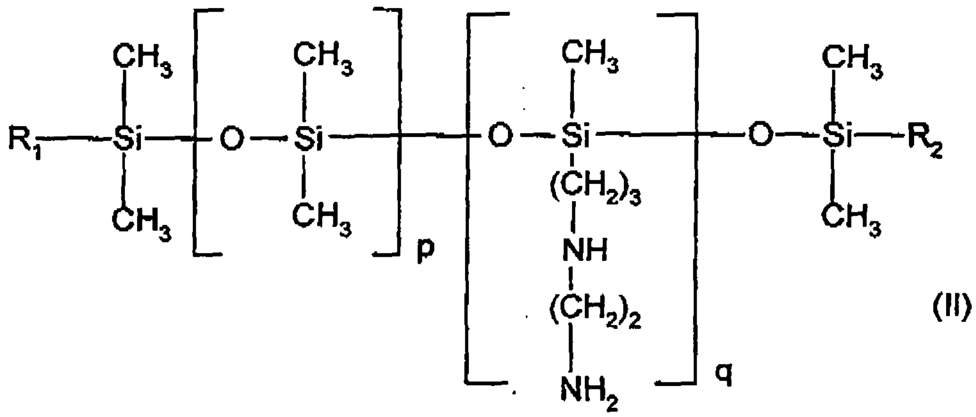

(II)

formule (II) dans laquelle :

p et q sont des nombres tels que la somme (p+q) varie de 1 à 1000 et en particulier de 50 à 350 et plus particulièrement de 150 à 250,
p désignant un nombre de 0 à 999 et notamment de 49 à 349 et plus particulièrement de 159 à 239 et q désignant un nombre de 1 à 1000, et notamment de 1 à 10 et plus particulièrement de 1 à 5 ;
$R_1$, $R_2$, différents, représentent un radical hydroxy ou alcoxy en $C_1$-$C_4$, l'un, au moins des radicaux $R_1$ ou $R_2$ désignant un radical alcoxy.

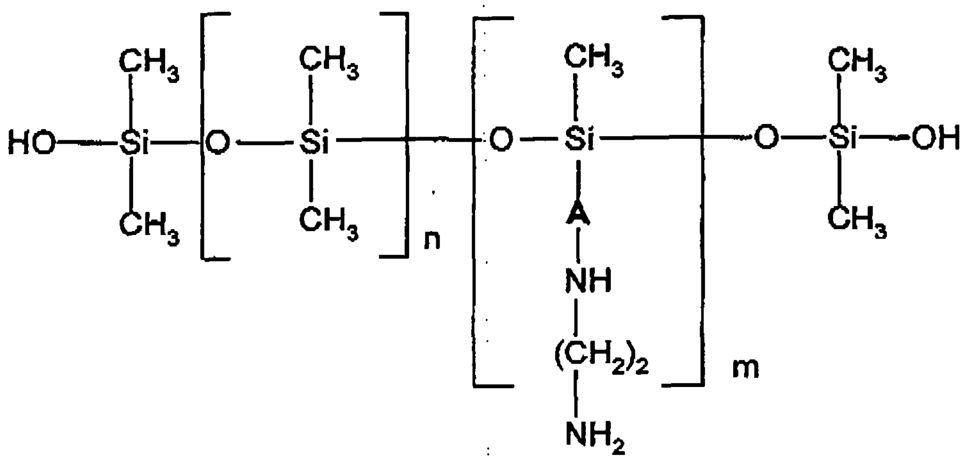

(III)

formule (III) dans laquelle :

A désigne un radical alkylène linéaire ou ramifié en $C_4$-$C_8$,
m et n sont des nombres tels que la somme (n+m) varie de 1 à 2000 et en particulier de 50 à 150, n désigne un nombre allant de 0 à 1999 et notamment de 49 à 149 et m pouvant désigner un nombre allant de 1 à 2000, et notamment de 1 à 10.

3. Composition selon la revendication 2, **caractérisée en ce que** le radical alcoxy en $C_1$-$C_4$ des formules (I) et (II) désigne le radical méthoxy.

4. Composition selon la revendication 2 ou 3, **caractérisée en ce que** pour les silicones aminées de formule (I), le rapport molaire hydroxy/alcoxy est compris entre 0,2 :1 et 0,4 :1 et de préférence entre 0,25 :1 et 0,35 :1 et plus particulièrement égal à 0,3.

5. Composition selon la revendication 2 ou 3, **caractérisée en ce que** pour les silicones aminées de formule (II), le rapport molaire hydroxy/alcoxy est compris entre 1 :0,8 et 1 :1,1 et de préférence entre 1 :0,9 et 1 : 1 et plus particulièrement est égal à 1 :0,95.

6. Composition selon l'une quelconque des revendications 1 à 4, **caractérisée en ce que** la silicone aminée de formule (I) présente une masse moléculaire moyenne en poids allant de 2000 à 1000000 et plus particulièrement de 3500 à 200000.

7. Composition selon l'une quelconque des revendications 1 à 3 et 5, **caractérisée en ce que** la silicone aminée de formule (II) présente une masse moléculaire moyenne en poids allant de 2000 à 200000, plus particulièrement de 5000 à 100000 et plus particulièrement encore de 10000 à 50000.

8. Composition selon la revendication 2, **caractérisée en ce que** dans la formule (III) A désigne un radical alkylène linéaire out ramifié en $C_4$.

9. Composition selon la revendication 1 ou 8, **caractérisée en ce que** la viscosité de la silicone aminée de formule (III) est supérieure à 25 000 $mm^2/s$ à 25°C.

**10.** Composition selon la revendication 9, **caractérisée en ce que** la viscosité de la silicone aminée de formule (III) va de 30 000 à 200 000 $mm^2/s$ à 25°C et plus préférentiellement de 30 000 à 150 000 $mm^2/s$ à 2S°C.

**11.** Composition selon l'une quelconque des revendications précédentes, **caractérisée en ce que** la silicone aminée est présente dans la composition en une quantité allant de 0,1 à 10%, et de préférence de 0,5 à 5% en poids du poids total de la composition.

**12.** Composition selon l'une quelconque des revendications précédentes **caractérisée en ce qu'**elle contient au moins un polymère associatif cationique choisi parmi les celluloses quaternisées et les hydroxyéthylcelluloses quaternisées, les groupes alkyle desdites celluloses ou hydroxyéthylcelluloses quaternisées comportant de 8 à 30 atomes de carbone.

**13.** Composition selon la revendication 12, **caractérisée en ce que** le polymère associatif cationique est une hydroxyéthylcellulose quaternisée modifiée par un groupement alkyle en $C_{12}$ ou $C_{18}$.

**14.** . Composition selon l'une quelconque des revendications 1 à 11, **caractérisée en ce qu'**elle contient au moins un polyuréthane cationique de formule (Ia) suivante:

$$R\text{-}X\text{-}(P)_n\text{-}[L\text{-}(Y)_m]_r\text{-}L'\text{-}(P')_p\text{-}X'\text{-}R' \qquad (Ia)$$

dans laquelle :

R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;

la molécule contenant au moins une fonction amine protonée ou quaternisée et au moins un groupement hydrophobe.

**15.** Composition selon l'une quelconque des revendications précédentes, **caractérisée en ce que** ledit ou lesdits polymères associatifs sont présents dans la composition à des teneurs en poids comprises entre 0,05 et 10%, et encore de préférence entre 0,1 et 5% du poids total de la composition.

**16.** Composition selon l'une quelconque des revendications précédentes, **caractérisée en ce que** le rapport en poids de la silicone aminée sur le polymère associatif est compris entre 1 et 10.

**17.** Composition selon l'une quelconque des revendications précédentes, **caractérisée en ce que** le colorant d'oxydation est choisi parmi les bases d'oxydation et/ou les coupleurs.

**18.** Composition selon la revendication 17, **caractérisée en ce qu'**elle comprend au moins une base d'oxydation.

**19.** Composition selon la revendication 17 ou 18, **caractérisée en ce que** les bases d'oxydation sont choisies parmi les ortho- et para-phénylènediamines, les bases doubles, les ortho- et para-aminophénols, les bases hétérocycliques, ainsi que les sels d'addition de ces composés avec un acide.

**20.** Composition selon la revendication 19, **caractérisée en ce que** les para-phenylenediamines sont choisies parmi les composés de structure (I) suivante :

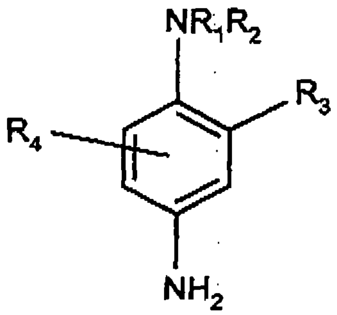

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

$R_1$ et $R_2$ peuvent également former avec l'atome d'azote qui les porte un hétérocycle azoté à 5 ou 6 chaînons éventuellement substitué par un

ou plusieurs groupements alkyle, hydroxy ou uréido :

- $R_3$ représente un atome d'hydrogène, un atome d'halogène, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoaolcoxy en $C_1$-$C_4$ ou carbamoylaminoalcoxy en $C_1$-$C_4$,
- $R_4$ représente un atome d'hydrogène, d'halogène ou un radical alkyle en $C_1$-$C_4$.

**21.** Composition selon la revendication 19, **caractérisée en ce que** les bases doubles sont choisies parmi les composés de structure (II) suivante :

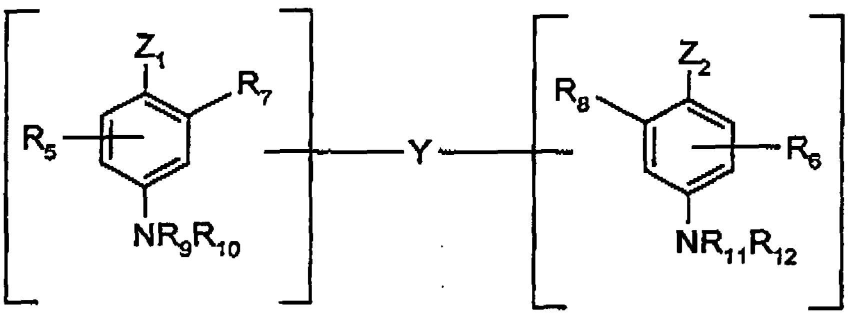

(II)

dans laquelle :

- $Z_1$ et $Z_2$, identiques ou différents, représentent un radical hydroxyle ou -$NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un plusieurs groupements azotés et/ou par un ou plusieurs hétéroatomes, et éventuellement substituée par un plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.

**22.** Composition selon la revendication 19, **caractérisée en ce que** les para-aminophénols sont choisis parmi les composés de structure (III) suivante :

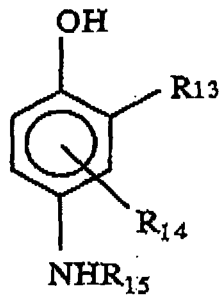

(III)

dans laquelle :

$R_{13}$ représente un atome d'hydrogène, un atome d'halogène, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl ($C_1$-$C_4$)aminoalkyle en $C_1$-$C_4$,
$R_{14}$ représente un atome d'hydrogène, un atome d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle ($C_1$-$C_4$),
$R_{15}$ représente un atome d'hydrogène ou un radical alkyle en $C_1$-$C_4$.

**23.** Composition selon la revendication 19, **caractérisée en ce que** les bases hétérocycliques sont choisies parmi les dérivés pyridiniques, les dérivés pyrimidiniques, les dérivés pyrazoliques.

**24.** Composition selon l'une quelconque des revendications 17 à 23, **caractérisée en ce que** les bases d'oxydation représentent de 0,0005 à 12%, et de préférence de 0,005 à 8% en poids du poids total de la composition.

**25.** Composition selon la revendication 17, **caractérisée en ce que** les coupleurs sont choisis parmi les métaphénylènediamines, les méta-aminophénols, les métadiphénols, les coupleurs hérérocycliques, et les sels d'addition de ces composés avec un acide.

**26.** Composition selon l'une quelconque des revendications 17 ou 25, **caractérisée en ce que** les coupleurs représentent de 0,0001, à 10%, et de préférence de 0,005 à 5% en poids du poids total de la composition.

**27.** Composition selon l'une quelconque des revendications 17 à 26, **caractérisée en ce** les sels d'addition avec un acide des colorants d'oxydation sont choisis parmi les chlorhydrates, les bromhydrates, les sulfates, les tartrates, les lactates et les acétates.

**28.** Composition selon l'une quelconque des revendications précédentes, **caractérisée en ce qu'**elle comprend en outre des colorants directs.

**29.** Composition selon l'une quelconque des revendications précédentes, **caractérisée en ce que** la composition contient en outre au moins un polymère substantif amphotère ou cationique non siliconé différent de ceux définis dans l'une quelconque des revendications 1 et 12 à 14.

**30.** Composition selon la revendication 29, **caractérisée en ce que** le polymère substantif est l'homopolymère de chlorure de diméthyldiallylammonium.

**31.** Composition selon la revendication 29, **caractérisée en ce que** le polymère substantif est un polymère de polyammonium, quaternaire constitué de motifs récurrents répondant à la formule (W) suivante :

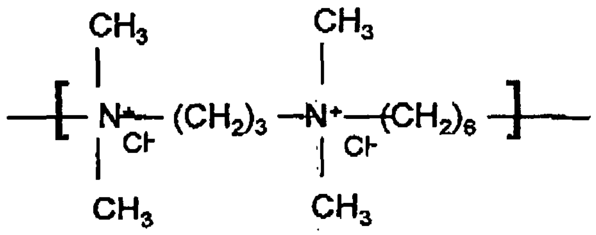 (W)

**32.** Composition selon la revendication 29, **caractérisée en ce que** le polymère substantif est un polymère de polyammonium quaternaire constitué de motifs récurrents répondant à la formule (U) suivante :

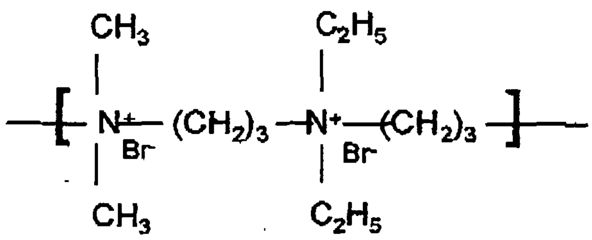 (U)

**33.** Composition selon l'une quelconque des revendications 29 à 32, **caractérisée en ce que** le ou les polymères substantifs cationiques ou amphotères représentent de 0,01 à 10%, de préférence de 0,05 à 5%, et encore de préférence de 0,1 à 3% du poids total de la composition.

**34.** Composition selon l'une quelconque des revendications précédentes, **caractérisée en ce qu'**elle comprend au moins un tensioactif choisi parmi les tensioactifs anioniques, amphotères, non ioniques, zwitterioniques et cationiques.

**35.** Composition selon la revendication 34, **caractérisée en ce que** le tensioactif est non ionique.

**36.** Composition selon la revendication 34 ou 35, **caractérisée en ce que** les tensioactifs représentent de 0,01 à 40% et de préférence de 0,5 à 30% en poids, du poids total de la composition.

**37.** Composition selon l'une quelconque des revendications précédentes, **caractérisée en ce qu'**elle comprend au moins un épaississant d'appoint.

**38.** Composition selon la revendication 37, **caractérisée en ce que** l'épaississant d'appoint est un épaississant cellulosique, un dérivé de gomme de guar, une gomme d'origine microbienne, un épaississant synthétique.

**39.** Composition selon la revendication 37 ou 38, **caractérisée en ce que** le ou les épaississants d'appoint représentent de 0,01 à 10% en poids du poids total de la composition.

**40.** Composition selon l'une quelconque des revendications précédentes, **caractérisée en ce qu'**elle comprend en outre au moins un agent réducteur, dans des quantités allant de 0,05 à 1,5% en poids par rapport au poids total de la composition.

**41.** Composition prête à l'emploi selon l'une quelconque des revendications précédentes, **caractérisée en ce qu'**elle comprend en outre un agent oxydant.

**42.** Composition selon la revendication 41, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

**43.** Composition selon la revendication 21, **caractérisée en ce que** l'agent oxydant est le peroxyde d'hydrogène.

**44.** Composition selon la revendication 43, **caractérisée en ce que** l'agent oxydant est une solution d'eau oxygénée dont le titre varie de 1 à 40 volumes.

**45.** Composition selon la revendication 44, **caractérisée en ce qu'**elle possède un pH allant de 4 à 11.

## Claims

**1.** Composition for the oxidation dyeing of keratin fibres, in particular of human keratin fibres, such as the hair, **characterized in that** it comprises, in a medium that is suitable for dyeing,

a) at least one oxidation dye,
b) at least one cationic associative polymer comprising at least one fatty chain, chosen from:

(i) quaternized celluloses modified with groups comprising at least one fatty chain,
(ii) quaternized hydroxyethylcelluloses modified with groups comprising at least one fatty chain, and
(iii) cationic polyurethanes, and

c) at least one aminosilicone,

the aminosilicone(s)/associative polymer(s) weight ratio being greater than 1.

**2.** Composition according to Claim 1, **characterized in that** the aminosilicone is of formula (I), (II) or (III) below:

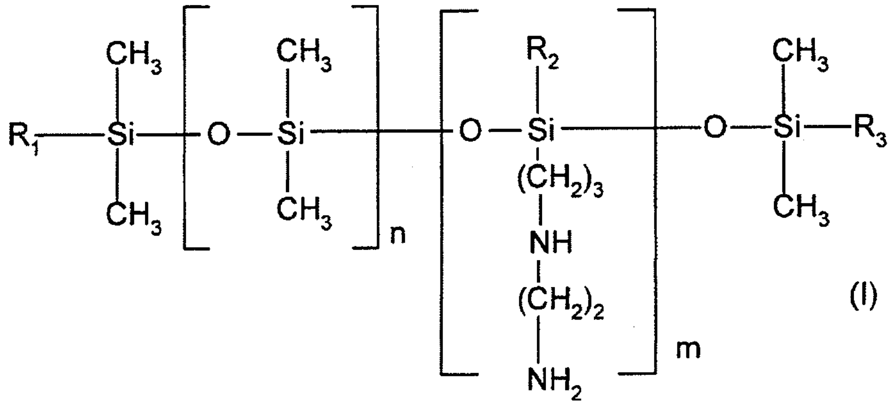

(I)

in which formula (I):

m and n are numbers such that the sum (n+m) can vary from 1 to 1 000, in particular from 50 to 250 and more particularly from 100 to 200,
n denotes a number from 0 to 999 and in particular from 49 to 249 and more particularly from 125 to 175 and m denotes a number from 1 to 1 000 and in particular from 1 to 10 and more particularly from 1 to 5;
$R_1$, $R_2$ and $R_3$, which are identical or different, represent a hydroxyl or a $C_1$-$C_4$ alkoxy radical, at least one of the radicals $R_1$ to $R_3$ denoting an alkoxy radical;

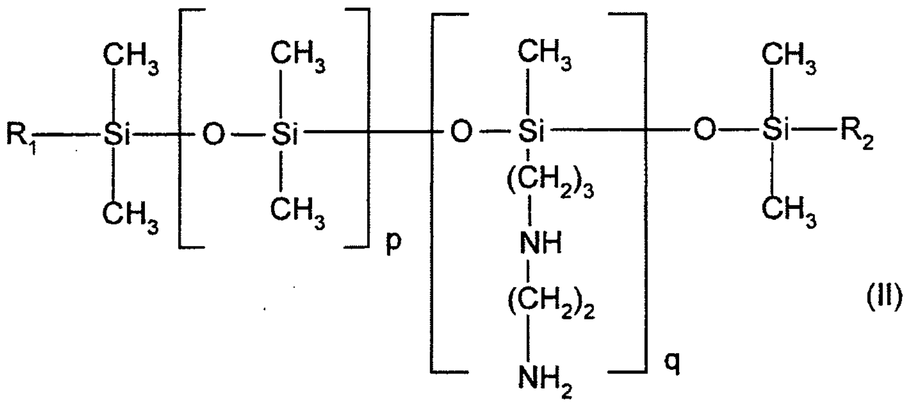

(II)

in which formula (II):

p and q are numbers such that the sum (p+q) can vary from 1 to 1 000, in particular from 50 to 350 and more particularly from 150 to 250,
p denotes a number from 0 to 999 and in particular from 49 to 349 and more particularly from 159 to 239 and q denotes a number from 1 to 1 000 and in particular from 1 to 10 and more particularly from 1 to 5;
$R_1$ and $R_2$, which are different, represent a hydroxyl or $C_1$-$C_4$ alkoxy radical, at least one of the radicals, $R_1$ or $R_2$, denoting an alkoxy radical;

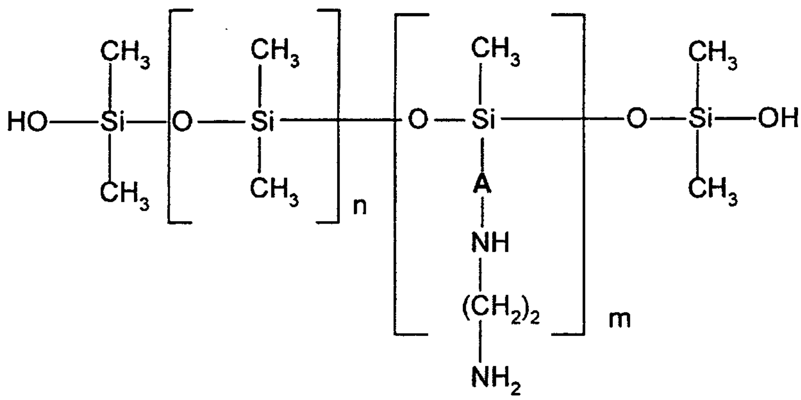

(III)

in which formula (III):

A denotes a linear or branched $C_4$-$C_8$ alkylene radical,
m and n are numbers such that the sum (n+m) can vary from 1 to 2 000, and in particular from 50 to 150,
n denotes a number ranging from 0 to 1999 and in particular from 49 to 149 and m denotes a number ranging from 1 to 2 000 and in particular from 1 to 10.

3. Composition according to Claim 2, **characterized in that** the $C_1$-$C_4$ alkoxy radical of formulae (I) and (II) denote the methoxy radical.

4. Composition according to Claim 2 or 3, **characterized in that**, for the aminosilicones of formula (I), the hydroxyl/alkoxy molar ratio is between 0.2:1 and 0.4:1 and preferably between 0.25:1 and 0.35:1 and more particularly is equal to 0.3.

5. Composition according to Claim 2 or 3, **characterized in that**, for the aminosilicones of formula (II), the hydroxyl/alkoxy molar ratio is between 1:0.8 and 1:1.1 and preferably between 1:0.9 and 1:1 and more particularly is equal to 1:0.95.

**6.** Composition according to any one of Claims 1 to 4, **characterized in that** the aminosilicone of formula (I) has a weight-average molecular mass ranging from 2 000 to 1 000 000 and more particularly from 3 500 to 200 000.

**7.** Composition according to any one of Claims 1 to 3 and 5, **characterized in that** the aminosilicone of formula (II) has a weight-average molecular mass ranging from 2 000 to 200 000, more particularly from 5 000 to 100 000 and more particularly still from 10 000 to 50 000.

**8.** Composition according to Claim 2, **characterized in that** in the formula (III) A denotes a linear or branched $C_4$ alkylene radical.

**9.** Composition according to Claim 1 or 8, **characterized in that that** the viscosity of the aminosilicone of formula (III) is greater than 25 000 $mm^2/s$ at 25°C.

**10.** Composition according to Claim 9, **characterized in that** the viscosity of the aminosilicone of formula (III) ranges from 30 000 to 200 000 $mm^2/s$ at 25°C and more preferably from 30 000 to 150 000 $mm^2/s$ at 25°C.

**11.** Composition according to any one of the preceding claims, **characterized in that** the aminosilicone is present in the composition in an amount ranging from 0.1% to 10% and preferably from 0.5% to 5% by weight, relative to the total weight of the composition.

**12.** Composition according to any one of the preceding Claims, **characterized in that** it comprises at least one cationic associative polymer chosen from quaternized celluloses and quaternized hydroxyethylcelluloses, the alkyl groups of the said quaternized celluloses or hydroxyethylcelluloses containing from 8 to 30 carbon atoms.

**13.** Composition according to 12, **characterized in that** the cationic associative polymer is a quaternized hydroxyethylcellulose modified with a $C_{12}$ or $C_{18}$ alkyl group.

**14.** Composition according to any one of Claims 1 to 11, **characterized in that** it comprises at least one cationic polyurethane of formula (Ia) below:

$$R\text{-}X\text{-}(P)_n\text{-}[L\text{-}(Y)_m]_r\text{-}L'\text{-}(P')_p\text{-}X'\text{-}R' \qquad \text{(Ia)}$$

in which:

R and R', which may be identical or different, represent a hydrophobic group or a hydrogen atom;
X and X', which may be identical or different, represent a group comprising an amine function optionally bearing a hydrophobic group, or alternatively the group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each amount, independently of each other, to between 0 and 1000;

the molecule containing at least one protonated or quaternized amine function and at least one hydrophobic group.

**15.** Composition according to any one of the preceding claims, **characterized in that** the said associative polymer or polymers are present in the composition in amounts by weight of between 0.05% and 10% and more preferably between 0.1% and 5% of the total weight of the composition.

**16.** Composition according to any one of the preceding claims, **characterized in that** the ratio by weight of the aminosilicone to the associative polymer is between 1 and 10.

**17.** Composition according to any one of the preceding claims, **characterized in that** the oxidation dye is chosen from oxidation bases and/or couplers.

**18.** Composition according to Claim 17, **characterized in that** it comprises at least one oxidation base.

**19.** Composition according to Claim 17 or 18, **characterized in that** the oxidation bases are chosen from ortho- and para-phenylenediamines, double bases, ortho- and para-aminophenols, and heterocyclic bases, and also the addition salts of these compounds with an acid.

**20.** Composition according to Claim 19, **characterized in that** the para-phenylenediamines are chosen from the compounds of formula (I) below:

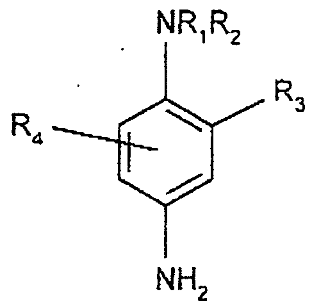

(I)

in which:

- $R_1$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical or a $C_1$-$C_4$ alkyl radical substituted with a nitrogenous, phenyl or 4'-aminophenyl group;
- $R_2$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$) alkoxy ($C_1$-$C_4$) alkyl radical or a $C_1$-$C_4$ alkyl radical substituted with a nitrogenous group;

$R_1$ and $R_2$ may also form, with the nitrogen atom that bears them, a 5- or 6-membered nitrogen heterocycle optionally substituted with one or more alkyl, hydroxyl or ureido groups;

- $R_3$ represents a hydrogen atom, a halogen atom, a $C_1$-$C_4$ alkyl radical, a sulpho radical, a carboxyl radical, a $C_1$-$C_4$ monohydroxyalkyl radical, a $C_1$-$C_4$ hydroxyalkoxy radical, an acetylamino($C_1$-$C_4$)alkoxy radical, a mesylamino($C_1$-$C_4$)alkoxy radical or a carbamoylamino($C_1$-$C_4$)alkoxy radical,
- $R_4$ represents a hydrogen or halogen atom or a $C_1$-$C_4$ alkyl radical.

**21.** Composition according to Claim 19, **characterized in that** the double bases are chosen from the compounds of structure (II) below:

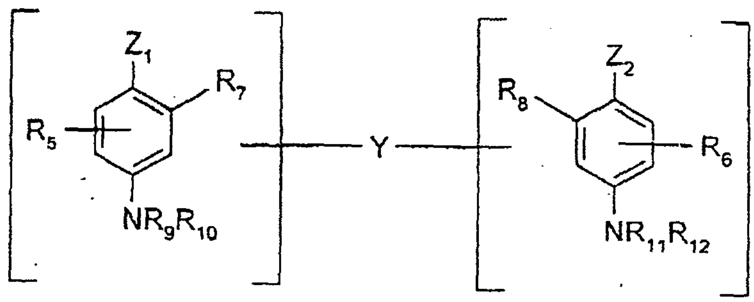

(II)

in which:

- $Z_1$ and $Z_2$, which may be identical or different, represent a hydroxyl or -$NH_2$ radical which may be substituted with a $C_1$-$C_4$ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms, and optionally substituted with one or more hydroxyl or $C_1$-$C_6$ alkoxy radicals;

- $R_5$ and $R_6$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a linker arm Y;
- $R_7$, $R_8$, $R_9$, $R_{10}$, $R_{11}$ and $R_{12}$, which may be identical or different, represent a hydrogen atom, a linker arm Y or a $C_1$-$C_4$ alkyl radical;

it being understood that the compounds of formula (II) contain only one linker arm Y per molecule.

**22.** Composition according to Claim 19, **characterized in that** the para-aminophenols are chosen from the compounds of structure (III) below:

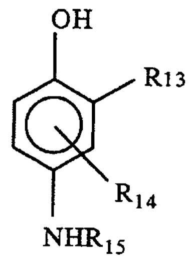

(III)

in which:

$R_{13}$ represents a hydrogen atom, a halogen atom, or a $C_1$-$C_4$ alkyl, $C_1$-$C_4$ monohydroxyalkyl, $(C_1$-$C_4)$alkoxy $(C_1$-$C_4)$-alkyl, $C_1$-$C_4$ aminoalkyl or hydroxy$(C_1$-$C_4)$alkylamino-$(C_1$-$C_4)$ alkyl radical,
$R_{14}$ represents a hydrogen atom, a halogen atom, or a $C_1$-$C_4$ alkyl, $C_1$-$C_4$ monohydroxyalkyl, $C_2$-$C_4$ polyhydroxyalkyl, $C_1$-$C_4$ aminoalkyl, $C_1$-$C_4$ cyanoalkyl or $(C_1$-$C_4)$ alkoxy$(C_1$-$C_4)$ alkyl radical.
$R_{15}$ represents a hydrogen atom or a $C_1$-$C_4$ alkyl radical.

**23.** Composition according to Claim 19, **characterized in that** the heterocyclic bases are chosen from pyridine derivatives, pyrimidine derivatives, and pyrazole derivatives.

**24.** Composition according to any one of Claims 17 to 23, **characterized in that** the oxidation bases represent from 0.0005% to 12% and preferably from 0.005% to 8% by weight relative to the total weight of the composition.

**25.** Composition according to Claim 17, **characterized in that** the couplers are chosen from meta-phenylenediamines, meta-aminophenols, meta-diphenols and heterocyclic couplers, and the addition salts of these compounds with an acid.

**26.** Composition according to either of Claims 17 or 25, **characterized in that** the couplers represent from 0.0001% to 10% and preferably from 0.005% to 5% by weight relative to the total weight of the composition.

**27.** Composition according to any one of Claims 17 to 26, **characterized in that** the addition salts with an acid of the oxidation dyes are chosen from hydrochlorides, hydrobromides, sulphates, tartrates, lactates and acetates.

**28.** Composition according to any one of the preceding claims, **characterized in that** it also comprises direct dyes.

**29.** Composition according to any one of the preceding claims, **characterized in that** the composition further comprises at least one amphoteric or cationic substantive non-silicon based polymer different from those defined in any one of Claims 1 and 12 to 14.

**30.** Composition according to Claim 29, **characterized in that** the substantive polymer is the homopolymer of dimethyldiallylammonium chloride.

**31.** Composition according to Claim 29, **characterized in that** the substantive polymer is a polymer of quaternary polyammonium comprising repeating units corresponding to formula (W) below:

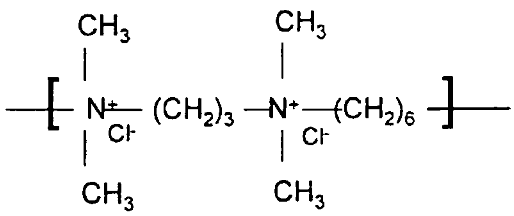

**(W)**

32. Composition according to Claim 29, **characterized in that** the substantive polymer is a polymer of quaternary polyammonium comprising repeating units corresponding to formula (U) below:

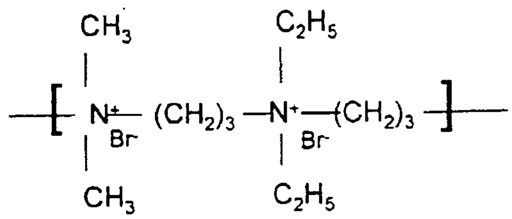

**(U)**

33. Composition according to any one of Claims 29 to 32, **characterized in that** the cationic or amphoteric substantive polymer or polymers represent from 0.01% to 10%, preferably from 0.05% to 5% and even more preferably from 0.1% to 3% of the total weight of the composition.

34. Composition according to any one of the preceding claims, **characterized in that** it comprises at least one surfactant chosen from anionic, amphoteric, non-ionic, zwitterionic and cationic surfactants.

35. Composition according to Claim 34, **characterized in that** the surfactant is non-ionic.

36. Composition according to Claim 34 or 35, **characterized in that** the surfactants represent from 0.01% to 40% and preferably from 0.5% to 30% by weight relative to the total weight of the composition.

37. Composition according to any one of the preceding claims, **characterized in that** it comprises at least one supplementary thickener.

38. Composition according to Claim 37, **characterized in that** the supplementary thickener is a cellulosic thickener, a guar gum derivative, a gum of microbial origin or a synthetic thickener.

39. Composition according to Claim 37 or 38, **characterized in that** the supplementary thickener or thickeners represent from 0.01% to 10% by weight relative to the total weight of the composition.

40. Composition according to any one of the preceding claims, **characterized in that** it also comprises at least one reducing agent, in amounts ranging from 0.05% to 1.5% by weight relative to the total weight of the composition.

41. Ready-to-use composition according to any one of the preceding claims, **characterized in that** it also comprises an oxidizing agent.

42. Composition according to Claim 41, **characterized in that** the oxidizing agent is chosen from hydrogen peroxide, urea peroxide, alkali metal bromates or ferricyanides, persalts, and redox enzymes together where appropriate with the respective donor or co-factor thereof.

43. Composition according to Claim 41, **characterized in that** the oxidizing agent is hydrogen peroxide.

44. Composition according to Claim 43, **characterized in that** the oxidizing agent is an aqueous hydrogen peroxide

solution whose titre ranges from 1 to 40 volumes.

**45.** Composition according to Claim 44, **characterized in that** it has a pH ranging from 4 to 11.

**Patentansprüche**

**1.** Zusammensetzung für die Oxidationsfärbung von Keratinfasern, insbesondere von Keratinfasern des Menschen, wie Haare, **gekennzeichnet dadurch, dass** sie in einem für die Färbung geeigneten Milieu umfasst:

a) mindestens ein Oxidationsfärbemittel,
b) mindestens ein assoziatives kationisches Polymer, das mindestens eine Fettkette aufweist, ausgewählt aus:

(i) quaternisierten Zellulosen, die nach Gruppen, die mindestens eine Fettkette aufweisen, modifiziert sind,
(ii) quaternisierten Hydroxyethyl-Zellulosen, die nach Gruppen, die mindestens eine Fettkette aufweisen, modifiziert sind, und
(iii) kationischen Polyethuranen, und

c) mindestens ein Amino-Silikon,

wobei das Gewichtsverhältnis der Amino-Silikone(n) zu dem (den) assoziative(n) Polymer(en) größer als 1 ist.

**2.** Zusammensetzung nach Anspruch 1, **gekennzeichnet dadurch, dass** das Amino-Silikon aus folgender Formel (I), (II) oder (III) besteht:

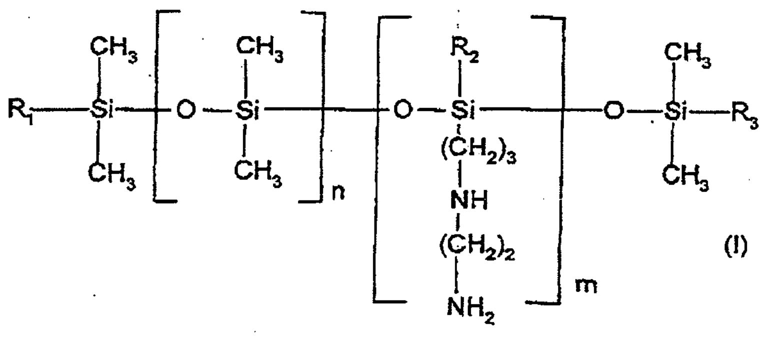

(I)

wobei in der Formel (I):

m und n Zahlen sind, deren Summe (n+m) sich zwischen 1 und 1000 bewegt und besonders zwischen 50 und 250 und ganz besonders zwischen 100 und 200;
n eine Zahl von 0 bis 999 bezeichnet und insbesondere zwischen 49 und 249 und ganz besonders zwischen 125 und 175 und m eine Zahl zwischen 1 und 1000 bezeichnet, und insbesondere zwischen 1 und 10 und ganz besonders zwischen 1 und 5;
$R_1$, $R_2$, $R_3$ identisch oder voneinander verschieden sind, wobei sie ein Hydroxy- oder Alkoxy-Radikal in $C_1$ bis $C_4$ bezeichnen, wobei mindestens eines der Radikale von $R_1$ bis $R_3$ ein Alkoxy-Radikal darstellt;

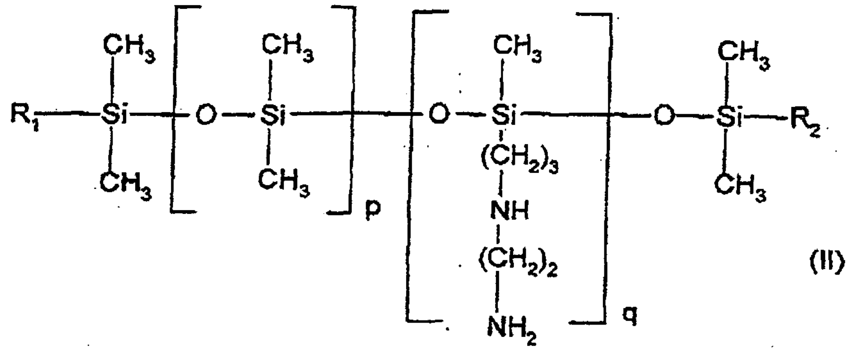

(II)

wobei in der Formel (II):

p und q solche Zahlen sind, dass die Summe (p+q) sich zwischen 1 und 1000 bewegt und besonders zwischen 50 und 350 und ganz besonders zwischen 150 und 250;
p eine Zahl von 0 bis 999 bezeichnet und insbesondere von 49 bis 349 und ganz besonders zwischen 159 und 239 und q eine Zahl zwischen 1 und 1000 bezeichnet, und insbesondere zwischen 1 und 10 und ganz besonders zwischen 1 und 5;
$R_1$ und $R_2$ voneinander verschieden sind, und ein Hydroxy- oder ein Alkoxy-Radikal in $C_1$ bis $C_4$ bezeichnen, wobei mindestens eines der Radikale $R_1$ oder $R_2$ ein Alkoxy-Radikal darstellt;

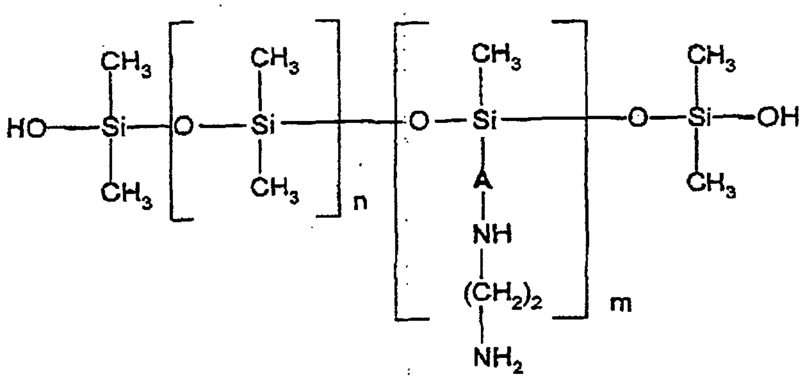

(III)

wobei in der Formel (III):

A ein Alkylen-Radikal in $C_4$ bis $C_8$ bezeichnet, das linear oder verzweigt ist,
m und n solche Zahlen sind, dass sich die Summe (n+m) zwischen 1 und 2000 bewegt und insbesondere zwischen 50 und 150, wobei n eine Zahl darstellt, die von 0 bis 1999 und insbesondere von 49 bis 149 geht und m eine Zahl bezeichnen kann, die von 1 bis 2000 geht und insbesondere von 1 bis 10.

3. Zusammensetzung nach Anspruch 2, **gekennzeichnet dadurch, dass** das Alkoxy-Radikal in $C_1$ bis $C_4$ der Formeln (I) und (II) das Methoxy-Radikal bezeichnet.

4. Zusammensetzung nach Anspruch 2 oder 3, **gekennzeichnet dadurch, dass** für die Amino-Silikone der Formel (I) das molare Verhältnis von Hydroxy / Alkoxy zwischen 0,2 : 1 und 0,4 : 1 und vorzugsweise zwischen 0,25 : 1 und 0,35 : 1 liegt und ganz besonders gleich 0,3 ist.

5. Zusammensetzung nach Anspruch 2 oder 3, **gekennzeichnet dadurch, dass** für die Amino-Silikone der Formel (II) das molare Verhältnis Hydroxy / Alkoxy zwischen 1 : 0,8 und 1 : 1,1 und vorzugsweise zwischen 1 : 0,9 und 1 : 1 liegt und ganz besonders gleich 1 : 0,95 ist.

6. Zusammensetzung nach irgendeinem der Ansprüche 1 bis 4, **gekennzeichnet dadurch, dass** das Amino-Silikon der Formel (I) eine im Gewicht mittlere molekulare Masse darstellt, die von 2000 bis 1.000.000 und ganz besonders von 3.500 bis 200.000 geht.

7. Zusammensetzung nach irgendeinem der Ansprüche 1 bis 3 und 5, **gekennzeichnet dadurch, dass** das Amino-Silikon der Formel (II) eine im Gewicht mittlere molekulare Masse darstellt, die von 2000 bis 200.000 geht, besonders von 5000 bis 100.000 und ganz besonders von 10.000 bis 50.000.

8. Zusammensetzung nach Anspruch 2, **gekennzeichnet dadurch, dass** das A in der Formel (III) ein Alkylen-Radikal in $C_4$ darstellt, dass linear oder verzweigt ist.

9. Zusammensetzung nach Anspruch 1 oder 8, **gekennzeichnet dadurch, dass** die Viskosität des Amino-Silikons der Formel (III) bei 25 °C über 25.000 $mm^2/s$ liegt.

10. Zusammensetzung nach Anspruch 9, **gekennzeichnet dadurch, dass** die Viskosität des Amino-Silikons der Formel (III) bei 25 °C von 30.000 bis 200.000 $mm^2/s$ geht und besonders bevorzugt bei 25°C von 30.000 bis 150.000 $mm^2/s$.

11. Zusammensetzung nach irgendeinem der vorangegangenen Ansprüche, **gekennzeichnet dadurch, dass** das Amino-Silikon, das in der Zusammensetzung vorhanden ist, eine Menge aufweist, die 0,1 bis 10 % und vorzugsweise 0,5 bis 5 % des Gewichtes vom Gesamtgewicht der Zusammensetzung beträgt.

12. Zusammensetzung nach irgendeinem der vorangegangenen Ansprüche, **gekennzeichnet dadurch, dass** sie mindestens ein kationisches assoziatives Polymer umfasst, dass aus den quaternisierten Zellulosen und den quaternisierten Hydroxyethyl-Zellulosen ausgewählt ist, umfasst, wobei die Alkyl-Gruppen der besagten quaternisierten Zellulosen oder Hydroxyethyl-Zellulosen 8 bis 30 Kohlenstoffatome aufweisen.

13. Zusammensetzung nach Anspruch 12, **gekennzeichnet dadurch, dass** das kationische assoziative Polymer eine quaternisierte Hydroxyethyl-Zellulose ist, die durch eine Alkyl-Gruppe in $C_{12}$ oder $C_{18}$ modifiziert ist.

14. Zusammensetzung nach irgendeinem der Ansprüche 1 bis 11, **gekennzeichnet dadurch, dass** diese mindestens ein kationisches Polyurethan der folgenden Formel (Ia) aufweist:

$$R\text{-}X\text{-}(P)_n\text{-}[L\text{-}(Y)_m]_r\text{-}L'\text{-}(P')_p\text{-}X'\text{-}R' \qquad \text{(Ia)}$$

wobei:

R und R' identisch oder voneinander verschieden sind und eine hydrophobische Gruppe oder ein Wasserstoffatom darstellen;
X und X' identisch oder voneinander verschieden sind und eine Gruppe darstellen, die eine Aminfunktion umfasst, welche eine hydrophobische Gruppe aufweist oder nicht, oder die Gruppe L";
L, L' und L" identisch oder voneinander verschieden sind und eine von einem Diisozyanat abgeleitete Gruppe darstellen;
P und P' identisch oder voneinander verschieden sind und eine Gruppe darstellen, die eine Aminfunktion umfasst, wobei diese eine hydrophobische Gruppe aufweist oder nicht;
Y eine hydrophile Gruppe darstellt;
r eine Ganzzahl zwischen 1 und 100 darstellt, vorzugsweise zwischen 1 und 50 und insbesondere zwischen 1 und 25,
n, m und p unabhängig voneinander einen Wert zwischen 0 und 1000 darstellen;
das Molekül mindestens eine protonierte oder quaternisierte Aminfunktion und mindestens eine hydrophobische Gruppe aufweist.

15. Zusammensetzung nach irgendeinem der vorangegangenen Ansprüche, **gekennzeichnet dadurch, dass** das oder die besagte(n) assoziative(n) Polymer(e) in der Zusammensetzung ein Gewicht aufweist / aufweisen, das zwischen 0,05 und 10 %, und besonders bevorzugt zwischen 0,1 und 5 % des Gesamtgewichtes der Zusammensetzung beträgt.

16. Zusammensetzung nach irgendeinem der vorangegangenen Ansprüche, **gekennzeichnet dadurch, dass** das Gewichtsverhältnis des Amino-Silikons zum assoziativen Polymer zwischen 1 und 10 beträgt.

**17.** Zusammensetzung nach irgendeinem der vorangegangenen Ansprüche, **gekennzeichnet dadurch, dass** das Oxidationsfärbemittel unter den Oxidationsbasen und / oder den Entwicklern ausgewählt ist.

**18.** Zusammensetzung nach Anspruch 17, **gekennzeichnet dadurch, dass** diese mindestens eine Oxidationsbase umfasst.

**19.** Zusammensetzung nach Anspruch 17 oder 18, **gekennzeichnet dadurch, dass** die Oxidationsbasen aus den Ortho- und Paraphenylendiaminen, den doppelten Basen, den Ortho- und Paraaminophenolen, den heterozyklischen Basen ausgewählt sind sowie auch unter den Zusatzsalzen dieser Verbindungen mit einer Säure.

**20.** Zusammensetzung nach Anspruch 19, **gekennzeichnet dadurch, dass** die Paraphenylendiamine aus den Verbindungen mit der folgenden Struktur (I) ausgewählt sind:

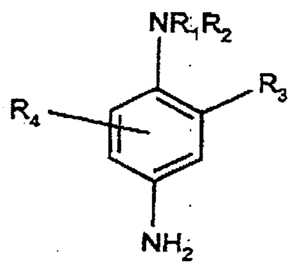

(I)

wobei:

- $R_1$ ein Wasserstoffatom, ein Alkyl-Radikal in $C_1$ bis $C_4$, ein Monohydroxyalkyl in $C_1$ bis $C_4$, ein Polyhydroxyalkyl in $C_2$ bis $C_4$, ein Alkoxy-($C_1$ bis $C_4$)Alkyl($C_1$ bis $C_4$), ein Alkyl in $C_1$ bis $C_4$ darstellt, dass durch eine azotierte Gruppe oder Phenyl oder 4'-Aminophenyl substituiert wird;
- $R_2$ ein Wasserstoffatom, ein Alkyl-Radikal in $C_1$ bis $C_4$, ein Monohydroxyalkyl in $C_1$ bis $C_4$ oder ein Polyhydroxyalkyl in $C_2$ bis $C_4$, ein Alkoxy-($C_1$ bis $C_4$)Alkyl($C_1$ bis $C_4$) oder ein Alkyl in $C_1$ bis $C_4$ darstellt, dass durch eine azotierte Gruppe substituiert wird;
- $R_1$ und $R_2$ mit dem azotierten Atom, das sie trägt, auch einen azotierten Heterozyklus mit 5 oder 6 Kettengliedern ausbilden können,

wobei dieser auch eventuell durch eine oder mehrere Alkyl-, Hydroxy- oder Ureidogruppen substituiert werden kann;

- $R_3$ ein Wasserstoffatom, ein Halogenatom, ein Alkyl-Radikal in $C_1$ bis $C_4$, Sulfo-, Carboxy-, Monohydroxyalkyl in $C_1$ bis $C_4$ oder ein Hydroxyalkoxy in $C_1$ bis $C_4$, ein Acetylaminoalkoxy in $C_1$ bis $C_4$, ein Mesylaminoalkoxy in $C_1$ bis $C_4$ oder ein Carbamoylaminoalkoxy in $C_1$ bis $C_4$ darstellt,
- $R_4$ ein Wasserstoffatom, ein Halogenatom oder ein Alkyl-Radikal in $C_1$ bis $C_4$ darstellt.

**21.** Zusammensetzung nach Anspruch 19, **gekennzeichnet dadurch, dass** die doppelten Basen aus den Verbindungen der folgenden Struktur (II) ausgewählt sind:

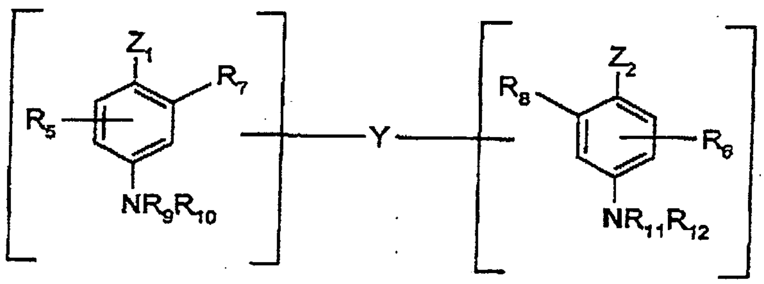

(II)

wobei:

- $Z_1$ und $Z_2$ identisch oder voneinander verschieden sind und ein Hydroxyl- oder $NH_2$-Radikal darstellen, wobei dieses durch ein Alkyl-Radikal in $C_1$ bis $C_4$ oder durch einen Verbindungsarm Y substituiert werden kann;
- der Verbindungsarm Y eine Alkylenkette darstellt, die 1 bis 14 Kohlenstoffatome umfasst, die linear oder verzweigt sind und durch mehrere azotische und / oder ein oder mehrere Heteroatome unterbrochen oder beendet werden können und eventuell durch mehrere Hydroxyl- oder Alkoxy-Radikale in $C_1$ bis $C_6$ substituiert werden können;
- $R_5$ und $R_6$ ein Wasserstoff- oder Halogenatom, ein Alkyl-Radikal in $C_1$ bis $C_4$, ein Monohydroxyalkyl in $C_1$ bis $C_4$ , ein Polyhydroxyalkyl in $C_2$ bis $C_4$, ein Aminoalkyl in $C_1$ bis $C_4$ oder einen Verbindungsarm Y darstellen;
- $R_7$, $R_8$, $R_9$, $R_{10}$, $R_{11}$ und $R_{12}$ identisch oder voneinander verschieden sind, wobei diese ein Wasserstoffatom darstellen, einen Verbindungsarm Y oder ein Alkyl-Radikal in $C_1$ bis $C_4$;

wobei vorausgesetzt wird, dass die Verbindungen der Formel (II) nur einen einzigen Verbindungsarm Y pro Molekül umfassen.

**22.** Zusammensetzung nach Anspruch 19, **gekennzeichnet dadurch, dass** die Paraaminophenole aus den Verbindungen der folgenden Struktur (III) ausgewählt sind:

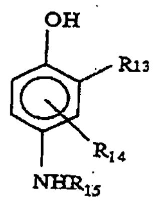

(III)

wobei:

$R_{13}$ ein Wasserstoffatom, ein Halogenatom, ein Alkyl-Radikal in $C_1$ bis $C_4$, ein Monohydroxyalkyl in $C_1$ bis $C_4$, ein Alkoxy($C_1$ bis $C_4$)Alkyl($C_1$ bis $C_4$) oder ein Aminoalkyl in $C_1$ bis $C_4$ oder ein Hydroxyalkyl ($C_1$ bis $C_4$)Aminoalkyl in $C_1$ bis $C_4$ darstellt;
$R_{14}$ ein Wasserstoffatom, ein Halogenatom, ein Alkyl-Radikal in $C_1$ bis $C_4$, ein Monohydroxyalkyl in $C_1$ bis $C_4$, ein Polyhydroxyalkyl in $C_2$ bis $C_4$, ein Aminoalkyl in $C_1$ bis $C_4$, ein Cyanoalkyl in $C_1$ bis $C_4$ oder ein Alkoxy($C_1$ bis $C_4$)Alkyl($C_1$ bis $C_4$) darstellt;
$R_{15}$ ein Wasserstoffatom oder ein Alkyl-Radikal in $C_1$ bis $C_4$ darstellt.

**23.** Zusammensetzung nach Anspruch 19, **gekennzeichnet dadurch, dass** die heterozyklischen Basen aus den pyridinischen Derivaten, den pyrimidinischen Derivaten oder den pyrazolischen Derivaten ausgewählt sind.

**24.** Zusammensetzung nach irgendeinem der Ansprüche 17 bis 23, **gekennzeichnet dadurch, dass** die Oxidationsbasen 0,0005 bis 12 % und vorzugsweise 0,005 bis 8 % an Gewicht in Bezug auf das Gesamtgewicht der Zusammensetzung darstellen.

**25.** Zusammensetzung nach Anspruch 17, **gekennzeichnet dadurch, dass** die Entwickler aus den Metaphenylendiaminen, den Metaaminophenolen, den Metadiphenolen, den heterozyklischen Entwicklern und den Zusatzsalzen dieser Verbindungen mit einer Säure ausgewählt sind.

**26.** Zusammensetzung nach irgendeinem der Ansprüche 17 bis 25, **gekennzeichnet dadurch, dass** die Entwickler 0,0001 bis 10 %, und vorzugsweise 0,005 bis 5 % an Gewicht in Bezug auf das Gesamtgewicht der Zusammensetzung ausmachen.

**27.** Zusammensetzung nach irgendeinem der Ansprüche 17 bis 26, **gekennzeichnet dadurch, dass** die Zusatzsalze mit einer Säure der Oxidationsfärbemittel aus den Chlorhydraten, den Bromhydraten, den Sulfaten, den Tartraten, den Lactaten und den Acetaten ausgewählt sind.

**28.** Zusammensetzung nach irgendeinem der vorangegangenen Ansprüche, **gekennzeichnet dadurch, dass** diese außerdem direkte Farbstoffe umfasst.

**29.** Zusammensetzung nach irgendeinem der vorangegangenen Ansprüche, **gekennzeichnet dadurch, dass** die Zusammensetzung außerdem mindestens ein amphoterisches oder kationisches substantives Polymer umfasst, das nicht silikonisiert ist und das sich von denen unterscheidet, die in irgendeinem der Ansprüche 1 und 12 bis 14 definiert sind.

**30.** Zusammensetzung nach Anspruch 29, **gekennzeichnet dadurch, dass** das substantive Polymer das Chlorid-Homopolymer aus Dimethyldiallylammonium ist.

**31.** Zusammensetzung nach Anspruch 29, **gekennzeichnet dadurch, dass** das substantive Polymer ein Polymer aus quaternisiertem Polyammonium ist, das aus sich wiederholenden Mustern zusammengesetzt ist und das der folgenden Formel (W) entspricht:

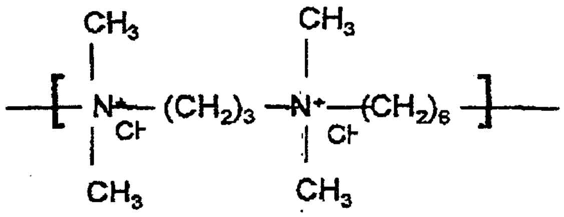

(W)

**32.** Zusammensetzung nach Anspruch 29, **gekennzeichnet dadurch, dass** das substantive Polymer ein Polymer aus quaternisiertem Polyammonium ist, das aus sich wiederholenden Mustern zusammengesetzt ist und das der folgenden Formel (U) entspricht:

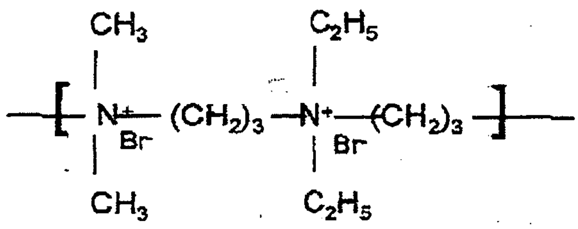

(U)

**33.** Zusammensetzung nach irgendeinem der Ansprüche 29 bis 32, **gekennzeichnet dadurch, dass** das oder die kationische(n) oder amphoterische(n) substantive (n) Polymer(e) 0,01 bis 10 %, vorzugsweise 0,05 bis 5 % und besonders bevorzugt 0,1 bis 3 % des Gesamtgewichtes der Zusammensetzung ausmachen.

**34.** Zusammensetzung nach irgendeinem der vorangegangenen Ansprüche, **gekennzeichnet dadurch, dass** diese mindestens ein Tensioaktiv umfasst, das aus den anionischen, amphoterischen, nichtionischen, zwitterionischen und kationischen Tensioaktiven ausgewählt ist.

**35.** Zusammensetzung nach Anspruch 34, **gekennzeichnet dadurch, dass** das Tensioaktiv nicht ionisch ist.

**36.** Zusammensetzung nach Anspruch 34 oder 35, **gekennzeichnet dadurch, dass** die Tensioaktive 0,01 bis 40 % und vorzugsweise 0,5 bis 30 % an Gewicht in Bezug auf das Gesamtgewicht der Zusammensetzung ausmachen.

**37.** Zusammensetzung nach irgendeinem der vorangegangenen Ansprüche, **gekennzeichnet dadurch, dass** diese mindestens ein unterstützendes Verdickungsmittel umfasst.

**38.** Zusammensetzung nach Anspruch 37, **gekennzeichnet dadurch, dass** das unterstützende Verdickungsmittel ein Verdickungsmittel aus Zellulose, ein Derivat aus Gummi Guar, ein Gummi mit mikrobiellem Ursprung, ein synthetisches Verdickungsmittel ist.

**39.** Zusammensetzung nach Anspruch 37 oder 38, **gekennzeichnet dadurch, dass** das oder die unterstützende(n) Verdickungsmittel 0,01 bis 10 % an Gewicht in Bezug auf das Gesamtgewicht der Zusammensetzung ausmachen.

**40.** Zusammensetzung nach irgendeinem der vorangegangenen Ansprüche, **gekennzeichnet dadurch, dass** diese außerdem mindestens einen reduzierenden Wirkstoff umfasst, wobei sich die Menge zwischen 0,05 und 1,5 % an Gewicht in Bezug auf das Gesamtgewicht der Zusammensetzung bewegt.

**41.** Zusammensetzung, die fertig zur Anwendung ist, gemäß irgendeinem der vorangegangenen Ansprüche, **gekennzeichnet dadurch, dass** diese außerdem einen oxidierenden Wirkstoff umfasst.

**42.** Zusammensetzung nach Anspruch 41, **gekennzeichnet dadurch, dass** der oxidierende Wirkstoff aus Hydrogenperoxid, Harnstoffperoxid, den Bromaten oder Eisencyanuren aus alkalinen Metallen, den Persalzen, den Oxydoreduktionsenzymen mit möglicherweise ihrem Geber oder Kofaktor ausgewählt ist.

**43.** Zusammensetzung nach Anspruch 41, **gekennzeichnet dadurch, dass** der oxidierende Wirkstoff Hydrogenperoxid ist.

**44.** Zusammensetzung nach Anspruch 43, **gekennzeichnet dadurch, dass** der oxidierende Wirkstoff eine Lösung aus Wasserstoffsuperoxyd ist, bei dem der Feingehalt zwischen 1 und 40 Volumina variiert.

**45.** Zusammensetzung nach Anspruch 44, **gekennzeichnet dadurch, dass** diese einen pH-Wert besitzt, der von 4 bis 11 geht.